# EUROPÄISCHE PATENTSCHRIFT

(11) **EP 3 325 122 B1**
(45) Veröffentlichungstag und Bekanntmachung des Hinweises auf die Patenterteilung: **05.02.2020**
(21) Anmeldenummer: 16736068.4
(22) Anmeldetag: 05.07.2016
(51) Int. Cl.: B01D 3/32, C07C 51/44, B08B 9/093

(54) **KOLONNE ZUR THERMISCHEN BEHANDLUNG VON FLUIDEN GEMISCHEN, INSBESONDERE SOLCHEN, DIE (METH)ACRYLMONOMERE ENTHALTEN**
COLUMN FOR THERMALLY TREATING FLUID MIXTURES, PARTICULARLY THOSE THAT CONTAIN (METH)ACRYLIC MONOMERS
COLONNE DE TRAITEMENT THERMIQUE DE MÉLANGES FLUIDES, CONTENANT NOTAMMENT DES MONOMÈRES (MÉTH)ACRYLIQUES

(30) Priorität: 17.07.2015 US 201562193611 P; 17.07.2015 DE 102015213493
(43) Veröffentlichungstag der Anmeldung: 30.05.2018
(73) Patentinhaber: BASF SE, 67056 Ludwigshafen (DE)
(72) Erfinder: HAMMON, Ulrich, 68163 Mannheim (DE); WALTER, Thomas, 67454 Hassloch (DE)
(74) Vertreter: BASF IP Association
(86) Internationale Anmeldenummer: PCT/EP2016/065773
(87) Internationale Veröffentlichungsnummer: WO 2017/012855

(56) Entgegenhaltungen:
- EP-A2- 1 561 521
- US-A1- 2011 240 458
- US-B1- 6 375 793
- US-B1- 6 409 886
- US-B1- 6 585 862

## Beschreibung

Die vorliegende Erfindung betrifft eine Kolonne zur thermischen Behandlung von fluiden Gemischen. Sie weist einen zylindrischen, vertikal ausgerichteten Kolonnenkörper auf, der einen Kolonnenhohlraum bildet. Die Kolonne umfasst ferner mehrere im Kolonnenhohlraum montierte Böden, die vertikal beabstandet voneinander angeordnet sind. Des Weiteren umfasst die Kolonne zumindest einen Stutzen, der in dem Kolonnenkörper angeordnet ist und der sich von dem Kolonnenkörper weg erstreckt, und eine verschließbare Revisionsöffnung, die bei dem Stutzen gebildet ist. Dabei ist in dem Kolonnenkörper eine Sprüheinrichtung angeordnet, mit der Flüssigkeit zumindest gegen die in den Kolonnenhohlraum gerichtete Oberfläche des Stutzens sprühbar ist. Die Sprüheinrichtung weist dabei eine Sprühdüse, eine Zuleitung und eine Sprühflüssigkeits-Zuführeinrichtung auf, wobei die Sprühflüssigkeits-Zuführeinrichtung ausgebildet ist, Sprühflüssigkeit dem Kolonnenhohlraum zu entnehmen, die entnommene Sprühflüssigkeit über die Zuleitung der Sprühdüse zuzuführen und mittels der Sprühdüse zumindest gegen die in den Kolonnenhohlraum gerichtete Oberfläche des Stutzens zu sprühen. Bei der Kolonne handelt es sich insbesondere um eine Trennkolonne. Des Weiteren betrifft die Erfindung ein thermisches Trennverfahren zwischen wenigstens einem in einer Kolonne aufsteigenden Gas und wenigstens einer in der Kolonne absteigenden Flüssigkeit.

In Trennkolonnen werden vielfach gasförmige (aufsteigend) und flüssige (absteigend) Stoffströme im Gegenstrom geführt, wobei wenigstens einer der Stoffströme insbesondere ein (Meth)acrylmonomer enthält. Infolge der zwischen den Stoffströmen bestehenden Ungleichgewichte findet ein Wärme- und Stoffaustausch statt, der letztlich die in der Trennkolonne gewünschte Abtrennung (bzw. Auftrennung) bedingt. In dieser Schrift sollen solche Trennverfahren als thermische Trennverfahren bezeichnet werden.

Beispiele für und damit Element der in dieser Schrift verwendeten Ausdrucksweise "thermische Trennverfahren" sind die fraktionierende Kondensation (vgl. z.B. DE 19924532 A1, DE 10243625 A1 und WO 2008/090190 A1) und die Rektifikation (bei beiden wird aufsteigende Dampfphase im Gegenstrom zu absteigender Flüssigphase geführt; die Trennwirkung beruht darauf, dass die Dampfzusammensetzung im Gleichgewicht von der Flüssigzusammensetzung verschieden ist), die Absorption (wenigstens ein aufsteigendes Gas wird zu wenigstens einer absteigenden Flüssigkeit im Gegenstrom geführt; die Trennwirkung beruht auf der unterschiedlichen Löslichkeit der Gasbestandteile in der Flüssigkeit) und die Desorption (der Umkehrprozess zur Ab sorption; das in der Flüssigphase gelöste Gas wird durch Partialdruckerniedrigung abgetrennt; erfolgt die Partialdruckerniedrigung des in der Flüssigphase Gelösten wenigstens teilweise dadurch, dass ein Trägergas durch die Flüssigphase geleitet wird, bezeichnet man dieses thermische Trennverfahren auch als Strippung; alternativ oder auch zusätzlich (zeitgleich als Kombination) kann die Partialdruckerniedrigung durch eine Absenkung des Arbeitsdruckes bewirkt werden).

Beispielsweise kann die Abtrennung von (Meth)acrylsäure bzw. (Meth)acrolein aus dem Produktgasgemisch der katalytischen Gasphasenoxidation so durchgeführt werden, dass die (Meth)acrylsäure bzw. das (Meth)acrolein durch Absorption in ein Lösungsmittel (z.B. Wasser oder ein organisches Lösungsmittel) oder durch fraktionierende Kondensation des Produktgasgemisches zunächst grundabgetrennt und das dabei anfallende Absorbat bzw. Kondensat nachfolgend unter Erhalt von mehr oder weniger reiner (Meth)acrylsäure bzw. (Meth)acrolein weiter aufgetrennt wird (vgl. z.B. DE-10332758 A1, DE 10243625 A1, WO 2008/090190 A1, DE 10336386 A1, DE 19924532 A1, DE 19924533 A1, DE 102010001228 A1, WO 2004/035514 A1, EP 1125912 A2, EP 982289 A2, EP 982287 A1 und DE 10218419 A1).

Die Schreibweise (Meth)acrylmonomere steht in dieser Schrift verkürzend für "Acrylmonomere und/oder Methacrylmonomere".

Der Begriff Acrylmonomere steht in dieser Schrift verkürzend für "Acrolein, Acrylsäure und/oder Ester der Acrylsäure".

Der Begriff Methacrylmonomere steht in dieser Schrift verkürzend für "Methacrolein, Methacrylsäure und/oder Ester der Methacrylsäure".

Im Besonderen sollen die in dieser Schrift angesprochenen (Meth)acrylmonomere die nachfolgenden (Meth)acrylsäureester umfassen: Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, isoButylmethacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, N,N-Dimethylaminoethylacrylat und N,N-Dimethylaminoethylmethacrylat.

(Meth)acrylmonomere sind wichtige Ausgangsverbindungen zur Herstellung von Polymerisaten, die z.B. als Klebstoffe oder als Wasser super absorbierende Materialien in Hygieneartikeln Verwendung finden.

Großtechnisch werden (Meth)acrolein und (Meth)acrylsäure vorwiegend durch katalytische Gasphasenoxidation geeigneter C₃-/C₄-Vorläuferverbindungen (oder von Vorläuferverbindungen derselben) hergestellt. Im Fall von Acrolein und Acrylsäure werden als solche Vorläuferverbindungen bevorzugt Propen und Propan verwendet. Im Fall der Methacrylsäure und des Methacroleins sind iso-Buten und iso-Butan die bevorzugten Vorläuferverbindungen.

Neben Propen, Propan, iso-Buten und iso-Butan eignen sich als Ausgangsstoffe jedoch auch andere 3 bzw. 4 Kohlenstoffatome enthaltende Verbindungen wie z.B. iso-Butanol, n-Propanol oder Vorläuferverbindungen derselben wie z.B. der Methylether von iso-Butanol. Acrylsäure kann auch durch gasphasenkatalytische Oxidation von Acrolein erzeugt werden. Methacrylsäure kann auch durch gasphasenkatalytische Oxidation von Methacrolein erzeugt werden.

Im Rahmen solcher Herstellverfahren werden normalerweise Produktgemische erhalten, aus welchen die (Meth)acrylsäure bzw. das (Meth)acrolein abgetrennt werden muss.

Ester der (Meth)acrylsäure sind z.B. durch direkte Umsetzung von (Meth)acrylsäure und/oder (Meth)acrolein mit den entsprechenden Alkoholen erhältlich. Allerdings fallen auch in diesem Fall zunächst Produktgemische an, aus denen die (Meth)acrylsäureester abgetrennt werden müssen.

Die Trennkolonnen, in denen diese Trennverfahren durchgeführt werden, enthalten trennwirksame Einbauten. Diese verfolgen bei den thermischen Trennverfahren den Zweck, die Oberfläche für den die Auftrennung in der Trennkolonne bewirkenden Wärme- und Stoffaustausch ("die Austauschfläche") zu erhöhen.

Als solche Einbauten kommen z.B. Packungen, Füllkörper und/oder Böden, die auch als Stoffaustauschböden bezeichnet werden, in Betracht. Häufig werden als Trennkolonnen solche verwendet, die wenigstens als einen Teil der trennwirksamen Einbauten wenigstens eine Abfolge von Stoffaustauschböden enthalten.

Stoffaustauschböden verfolgen den Zweck, in der Trennkolonne in Form von auf ihnen sich ausbildenden Flüssigkeitsschichten Gebiete mit im Wesentlichen geschlossenen flüssigen Phasen zur Verfügung zu stellen. Die Oberfläche des in der Flüssigkeitsschicht aufsteigenden und sich dabei in der flüssigen Phase verteilenden Dampf- bzw. Gasstroms ist dann die maßgebliche Austauschfläche.

Unter einer Abfolge von Stoffaustauschböden wird dabei eine Aufeinanderfolge (ein Nacheinander) von wenigstens zwei in der Trennkolonne übereinander angeordneten, im Regelfall baugleichen (d.h., identischen), Stoffaustauschböden verstanden. Anwendungstechnisch vorteilhaft ist der lichte Abstand zwischen zwei in einer solchen Serie (Reihe) von Stoffaustauschböden unmittelbar aufeinanderfolgenden Stoffaustauschböden einheitlich gestaltet (d.h., die Stoffaustauschböden sind in der Trennkolonne äquidistant übereinander angeordnet).

Die einfachste Ausführungsform eines Stoffaustauschbodens ist der sogenannte Regensiebboden. Dabei handelt es sich um eine Platte bzw. um zu einer Platte zusammengefügte Plattensegmente, die für die aufsteigende Gas- bzw. Dampfphase (die Begriffe "gasförmig" und "dampfförmig" werden in dieser Schrift synonym verwendet) über die Platte verteilte und im Wesentlichen plane Durchtrittsöffnungen, z.B. runde Löcher und/oder Schlitze, aufweist (vgl. z.B. DE 10230219 A1, EP 1279429 A1, US-A 3988213 und EP 1029573 A1). Darüber hinausgehende Öffnungen (z.B. wenigstens einen Ablaufschacht (wenigstens ein Ablaufsegment)) weisen Regensiebböden nicht auf. Durch diese Abwesenheit von Ablaufschächten müssen sich sowohl das in der Trennkolonne aufsteigende Gas (der in der Trennkolonne aufsteigende Dampf) als auch die in der Trennkolonne absteigende Flüssigkeit entgegengesetzt strömend im zeitlichen Wechsel durch die (gleichen) Durchtrittsöffnungen (durch die offenen Querschnitte der Durchtrittstellen) bewegen. Man spricht auch vom "dual-flow" von aufsteigendem Gas und absteigender Flüssigkeit durch die Durchtrittsöffnungen, weshalb in der Literatur für solche Stoffaustauschböden häufig auch der Begriff "Dual-Flow-Böden" verwendet wird.

Der Querschnitt der Durchtrittsöffnungen eines Dual-Flow-Bodens wird in an sich bekannter Weise seiner Belastung angepasst. Ist er zu klein, strömt das aufsteigende Gas mit so hoher Geschwindigkeit durch die Durchtrittsöffnungen, dass die in der Trennkolonne absteigende Flüssigkeit im Wesentlichen ohne Trennwirkung mitgerissen wird. Ist der Querschnitt der Durchtrittsöffnungen zu groß, bewegen sich aufsteigendes Gas und absteigende Flüssigkeit im Wesentlichen ohne Austausch aneinander vorbei und der Stoffaustauschboden läuft Gefahr trocken zu laufen.

D.h., der trennwirksame Arbeitsbereich eines Regensiebbodens (Dual-Flow-Boden) weist zwei Grenzen auf. Eine minimale Grenzgeschwindigkeit des aufsteigenden Gases muss gegeben sein, damit auf dem Regensiebboden eine gewisse Flüssigkeitsschicht gehalten wird, um ein trennwirksames Arbeiten des Regensiebbodens zu ermöglichen. Die obere Grenze der Geschwindigkeit des aufsteigenden Gases ist durch den Flutpunkt festgelegt, wenn die Gasgeschwindigkeit zum Stau der Flüssigkeit auf dem Regensiebboden führt und ihr Durchregnen verhindert wird.

Die Längstausdehnung der Durchtrittsöffnungen eines technischen Dual-Flow-Bodens (= längste direkte Verbindungslinie zweier auf der Umrisslinie des Querschnitts der Durchtrittsöffnung liegender Punkte) beträgt in typischer Weise 10 bis 80 mm (vgl. z.B. DE 10156988 A1). Normalerweise sind die Durchtrittsöffnungen innerhalb eines Regensiebbodens identisch (d.h., sie weisen alle die gleiche geometrische Form und den gleichen Querschnitt (die gleiche Querschnittsfläche) auf). Anwendungstechnisch zweckmäßig handelt es sich bei ihren Querschnittsflächen um Kreise. D.h., bevorzugte Durchtrittsöffnungen von Regensiebböden sind kreisförmige Bohrungen. Die Relativanordnung der Durchtrittsöffnungen eines Regensiebbodens folgt vorteilhaft einer strengen Dreiecksteilung (vgl. z.B. DE 10230219 A1). Selbstverständlich können die Durchtrittsöffnungen innerhalb ein und desselben Regensiebbodens auch unterschiedlich gestaltet sein (über den Regensiebboden variieren).

Anwendungstechnisch vorteilhaft umfasst eine Abfolge von Regensiebböden in einer Trennkolonne baugleiche (identische) Regensiebböden, die vorzugsweise äquidistant übereinander angeordnet sind.

Gemäß der DE 10156988 A1 können aber auch Abfolgen von Regensiebböden in Trennkolonnen zur Anwendung kommen, deren Querschnitt (bevorzugt kreisförmig) innerhalb eines Dual-Flow-Bodens zwar einheitlich gestaltet ist, innerhalb der Abfolge jedoch variiert (z.B. von unten nach oben abnimmt).

In der Regel schließt jeder Dual-Flow-Boden einer entsprechenden Bodenabfolge mit der Wand der Trennkolonne bündig ab. Es gibt aber auch Ausführungsvarianten, bei denen zwischen Kolonnenwand und Boden ein Zwischenraum besteht, der nur teilweise durch Brücken unterbrochen ist. Neben den eigentlichen Durchtrittsöffnungen weist ein Regensiebboden üblicherweise allenfalls noch Öffnungen auf, die der Befestigung des Bodens auf Auflageringen oder ähnlichem dienen (vgl. z.B. DE 10159823 A1).

Im normalen Arbeitsbereich einer Abfolge von Regensiebböden regnet die in der Trennkolonne absteigende Flüssigkeit in Tropfen von Dual-Flow-Boden zu Dual-Flow-Boden, d.h., die zwischen den Dual-Flow-Böden aufsteigende Gasphase wird von einer zerteilten Flüssigkeitsphase durchsetzt. Die auf dem jeweils unteren Regensiebboden auftreffenden Tropfen werden beim Auftreffen teilweise versprüht. Der durch die Durchtrittsöffnungen strömende Gasstrom sprudelt durch die auf der Oberfläche des Bodens gebildete Flüssigkeitsschicht, wobei ein intensiver Stoff- und Wärmeaustausch zwischen der Flüssigkeit und dem Gas stattfindet.

Je nach Flüssigkeits- und Gasbelastung neigen Regensiebböden bei Kolonnendurchmesser von >2 m dazu, dass sich geringe Ungleichverteilungen von Flüssigkeiten aufschaukeln können und so der Flüssigkeitsholdup eines Bodens großflächig schwankt bzw. sich eine umlaufende Welle ausbilden kann, was zum einen die mechanische Stabilität des Kolonnenkörpers negativ beeinflussen kann und zum anderen die Trennwirkung vermindert, da die Flüssigkeitsverteilung unter diesen Bedingungen dann zeitlich und örtlich stark unterschiedlich ist. Zur Vermeiden solcher Instationaritäten hat es sich deshalb als vorteilhaft erwiesen Strömungsbrecher in Form von senkrecht stehenden Blechen auf dem Bodenquerschnitt zu verteilen, die ein Aufschaukeln der Flüssigkeit innerhalb des Kolonnenkörpers verhindern oder zumindest stark reduzieren. Die Höhe der Bleche sollte dabei in etwa der Höhe der sich ausbildenden Flüssigkeitssprudelschicht entsprechen. Diese beträgt bei üblichen Belastungen ca. 20 cm.

Der Querschnitt einer Trennkolonne ist in der Regel kreisförmig. Dies trifft in entsprechender Weise auf die zugehörigen Stoffaustauschböden zu.

Für die Zwecke dieser Schrift verwendbare Dual-Flow-Böden sind z.B. in Technische Fortschrittsberichte, Bd. 61, Grundlagen der Dimensionierung von Kolonnenböden, Seite 198 bis 211, Verlag Theodor Steinkopf, Dresden (1967) und in der DE 10230219 A1 beschrieben.

Von der vorstehend beschriebenen Abfolge von Regensiebböden, die Stoffaustauschböden ohne Zwangsführung der auf den Boden absteigenden Flüssigkeit auf dem Boden umfasst, werden Abfolgen von Stoffaustauschböden mit einer solchen Flüssigkeitszwangsführung unterschieden.

Diese Stoffaustauschböden sind dadurch gekennzeichnet, dass sie neben den bereits beschriebenen Durchtrittsöffnungen zusätzlich wenigstens einen Ablaufschacht aufweisen. Dabei handelt es sich um wenigstens eine im Stoffaustauschboden befindliche Ablauföffnung, der die auf den Stoffaustauschboden abgestiegene Flüssigkeit (z.B. über ein Ablaufwehr (dieses kann in einfachster Ausführungsform eine Verlängerung der Ablauföffnung mit einem Hals (einem Kamin; im Fall einer kreisförmigen Ablauföffnung einer Röhre) nach oben sein)) zufließt, und die in einen zum in der Abfolge darunter liegenden Stoffaustauschboden zulaufenden Schacht ausläuft, der in der Regel zu einer in Kolonnenlängsrichtung weisenden Achse zentralsymmetrisch ausgebildet ist. Der Querschnitt des Schachts kann entlang dieser Achse variieren (sich z.B. verjüngen) oder auch konstant sein.

Durch den wenigstens einen Ablaufschacht des Stoffaustauschbodens kann innerhalb einer Abfolge derartiger Stoffaustauschböden die von einem höher gelegenen Stoffaustauschboden absteigende Flüssigkeit unabhängig vom nach wie vor durch die Durchtrittsöffnungen dieses Stoffaustauschbodens aufsteigenden Gas bzw. Dampf als wenigstens ein Zulauf an Flüssigkeit auf den nächst tiefer gelegenen Stoffaustauschboden der Abfolge absteigen.

Wesentliche Grundlage für diese Auftrennung der Strömungswege von absteigender Flüssigkeit und aufsteigendem Gas ist der hydraulische Verschluss (der Flüssigkeitsverschluss oder auch Schachtverschluss) des jeweiligen Ablaufschachtes für das aufsteigende Gas (ein Ablaufschacht darf für das aufsteigende Gas keinen Bypass an den Durchtrittsöffnungen vorbei bilden; der Gasstrom (der Dampfstrom) darf nicht durch einen Ablaufschacht an den Durchtrittsöffnungen vorbei aufsteigen).

Ein solcher hydraulischer Verschluss kann z.B. dadurch erreicht werden, dass man den Ablaufschacht so weit nach unten zieht (so weit nach unten auslaufen lässt), dass er tief genug in die auf dem nächst tiefer gelegenen Stoffaustauschboden der Abfolge befindliche Flüssigkeitsschicht eintaucht (ein solcher Verschluss wird in dieser Schrift auch als "statischer Verschluss" bezeichnet). Der hierfür notwendige Flüssigkeitsstand kann auf dem tiefer gelegenen Stoffaustauschboden z.B. durch die Höhe entsprechender Ablaufwehre gewährleistet werden.

Eine derartige Ausführung hat jedoch den Nachteil, dass der Bereich des tiefer gelegenen Stoffaustauschbodens, der sich unmittelbar unterhalb des Auslaufquerschnitts eines Ablaufschachtes des darüber befindlichen Stoffaustauschbodens (die sogenannte Zulauffläche) befindet, keine Durchtrittsöffnungen für das aufsteigende Gas aufweisen kann und so nicht für den Stoff- und Wärmeaustausch zwischen der auf dem tiefer gelegenen Stoffaustauschboden ausgebildeten Flüssigkeitsschicht und dem aufsteigenden Gas zur Verfügung steht.

Bei einer alternativen Ausführungsform ist das untere Auslaufende des Ablaufschachtes so weit hochgezogen, dass es nicht mehr in die auf dem darunter liegenden Stoffaustauschboden befindliche Flüssigkeitsschicht eintaucht. In diesem Fall verbleibt zwischen dem unteren Ende des wenigstens einen Ablaufschachts und dem Stoffaustauschboden, auf den der Ablaufschacht zuläuft, ein ausreichend großer Zwischenraum, in dem sich eine Sprudelschicht ausbilden und ein Stoff- und Wärmeaustausch zwischen einer (auf dem unteren Stoffaustauschboden) auflaufenden Flüssigkeitsschicht und einem (durch diesen Boden) aufsteigenden Gas stattfinden kann. D.h., in diesem Fall kann auch die "Zulauffläche" des wenigstens einen Ablaufschachtes auf dem darunter befindlichen Stoffaustauschboden Durchtrittsöffnungen aufweisen und so die verfügbare Austauschfläche des Stoffaustauschbodens, und damit seine Trennwirkung vergrößert werden.

Ein statischer Flüssigkeitsverschluss des Ablaufschachtes kann in diesem Fall z.B. mit Hilfe einer unter dem Auslaufende des Ablaufschachtes angebrachten Auffangtasse bewirkt werden. Anwendungstechnisch zweckmäßig wird in diesem Fall die Mantelwand der Auffangtasse so weit hochgezogen, dass das Auslaufende des Ablaufschachts in die Auffangtasse eintaucht (es ist auch möglich, die Unterkante des Ablaufschachts an der Oberkante der Auffangtasse enden zu lassen). Beim Betrieb der Kolonne sammelt sich in der Auffangtasse die durch den Ablaufschacht herabströmende Flüssigkeit so lange, bis diese über die Oberkante der Mantelwand der Auffangtasse abfließt. Die Unterkante des Ablaufschachts taucht in die in der Auffangtasse befindliche Flüssigkeit ein und die Auffangtasse bildet einen siphonartigen Flüssigkeitsverschluss des Ablaufschachtes.

Alternativ kann ein hochgezogener Ablaufschacht auch dynamisch verschlossen werden. Hierzu kann der Ablaufschacht z.B. an seinem unteren Ende mit einem Boden verschlossen werden, der mit Austrittsöffnungen versehen ist, die so dimensioniert sind, dass die Flüssigkeit im Ablaufschacht aufgestaut und das Eindringen von Gas verhindert wird (vgl. z.B. EP 0882481 A1 und DE 10257915 A1). Der Schachtverschluss wird in diesem Fall dynamisch durch den Druckverlust, der an den Austrittsöffnungen entsteht, hergestellt. D.h., beim statischen Verschluss erfolgt der Verschluss des Ablaufschachtes dadurch, dass dessen Auslaufende in gestaute Flüssigkeit eintaucht, und beim dynamischen Verschluss bewirken konstruktive Merkmale am Auslaufende des Ablaufschachts, dass die austretende (auslaufende) Flüssigkeit einen Druckverlust erleidet, der im Ablaufschacht einen Rückstau der in selbigem absteigenden Flüssigkeit bewirkt, welcher den Verschluss bedingt. Im einfachsten Fall kann ein solcher Druckverlust dadurch verursacht werden, dass man den Querschnitt der Austrittsöffnung des Ablaufschachts im Vergleich zum mittleren Querschnitt des Schachts klein wählt.

Für ein trennwirksames Arbeiten einer Abfolge von derartigen Stoffaustauschböden ist die Ausführung des wenigstens einen Ablaufschachtes relevant. Einerseits muss der Querschnitt des wenigstens einen Ablaufschachts hinreichend groß gewählt werden (in der Regel ist die entsprechende Querschnittsfläche größer als die Querschnittsfläche einer Durchtrittsöffnung), damit die Flüssigkeit auch bei der maximalen Belastung der Trennkolonne mit selbiger noch sicher durch den wenigstens einen Ablaufschacht absteigen kann und nicht bis auf den darüber liegenden Boden zurückstaut. Auf der anderen Seite muss sichergestellt werden, dass auch bei minimaler Flüssigkeitsbelastung der hydraulische Verschluss des wenigstens einen Ablaufschachtes noch besteht.

Bei geringer Gasbelastung besteht ebenfalls die Gefahr eines Durchregnens von Flüssigkeit durch die Durchtrittsöffnungen. Darüber hinaus muss sich die Flüssigkeit in einem Ablaufschacht so weit aufstauen können, bis das Gewicht der gestauten Flüssigkeitssäule ausreicht, um die Flüssigkeit in den Gasraum unterhalb des zum Ablaufschachts gehörigen Stoffaustauschbodens zu befördern. Diese Rückstauhöhe bestimmt die erforderliche Mindestlänge des Ablaufschachts und bestimmt so den in einer Abfolge entsprechender Stoffaustauschböden erforderlichen Bodenabstand mit. Wesentlicher Mitbestimmungsfaktor für vorstehende Rückstauhöhe (Rückstaulänge) ist der Druckverlust ΔP eines Stoffaustauschbodens. Diesen Druckverlust erleidet das aufsteigende Gas beim Durchströmen der Durchtrittsöffnungen sowie der "hydrostatischen" Höhe der Sprudelschicht auf dem Stoffaustauschboden. Er ist dafür verantwortlich, dass der Druck in der Gasphase einer Abfolge solcher Stoffaustauschböden von oben nach unten zunimmt. Für den "hydrostatischen" Druck hₚ der im Ablaufschacht gestauten Flüssigkeit eines Stoffaustauschbodens muss daher wenigstens die Bedingung hₚ > ΔP des Stoffaustauschbodens erfüllt sein. Diese Zusammenhänge sind dem Fachmann z.B. aus der EP 1704906 A1 ebenso bekannt, wie die Möglichkeit, mit einem Zulaufwehr auf dem tiefer liegenden Stoffaustauschboden sicherzustellen, dass bei statischem Verschluss des Ablaufschachtes des oberhalb gelegenen Stoffaustauschbodens in der Flüssigkeitsschicht auf dem tiefer liegenden Stoffaustauschboden, der Schachtverschluss auch bei geringer Belastung mit absteigender Flüssigkeit noch besteht. Allerdings erhöht die Mitverwendung eines Zulaufwehrs die Rückstauhöhe, die im Ablaufschacht erforderlich ist, um die in selbigem gestaute Flüssigkeit auf den tiefer gelegenen Stoffaustauschboden zu drücken. Insgesamt ermöglicht das Element des Ablaufschachts eine Verbreiterung des trennwirksamen Arbeitsbereichs im Vergleich zum Regensiebboden. Eine günstige Ablaufgeschwindigkeit der im Ablaufschacht gestauten Flüssigkeit aus dem Ablaufschacht heraus beträgt beim erfindungsgemäßen Verfahren z.B. 1,2 m/s.

Zusätzlich ermöglicht es eine Zwangsführung der auf einen Stoffaustauschboden absteigenden Flüssigkeit auf diesem Boden.

Weist z. B. nur eine Hälfte eines (vorzugsweise kreisförmigen) Stoffaustauschbodens wenigstens einen Ablaufschacht auf (d.h., alle Ablauföffnungen befinden sich mit ihrem vollen Umfang innerhalb des entsprechenden Kreissegments) und sind in einer Abfolge von wenigstens zwei baugleichen derartigen Stoffaustauschböden die Stoffaustauschböden in einer Trennkolonne so übereinander angeordnet, dass zwei von oben nach unten aufeinanderfolgende Stoffaustauschböden in der Trennkolonne jeweils um 180° um die Kolonnenlängsachse gegeneinander verdreht (gedreht) angebracht sind, so dass sich ihre Ablaufschächte auf einander gegenüber liegenden Seiten (in einander gegenüberliegenden Hälften) der Trennkolonne befinden, so muss sich die von einem oberen Stoffaustauschboden durch seinen wenigstens einen Ablaufschacht auf den darunter angebrachten Stoffaustauschboden absteigende Flüssigkeit auf diesem unteren Stoffaustauschboden über den unteren Stoffaustauschboden betrachtet von der wenigstens einen Zulauffläche des wenigstens einen Ablaufschachts des oberen (des darüber angebrachten) Stoffaustauschbodens (von dem wenigstens einen Zulauf durch den wenigstens einen Ablaufschacht des oberen Stoffaustauschbodens) in notwendiger Weise (d. h., gezwungenermaßen) zu dem wenigstens einen Ablaufschacht dieses unteren Stoffaustauschbodens strömen. D. h., die vom oberen auf den unteren Boden absteigende Flüssigkeit wird zwangsweise quer über den Boden von dem wenigstens einen Zulauf zu dem wenigstens einen Ablauf geführt.

Eine solche Flüssigkeitsführung auf einem Stoffaustauschboden innerhalb einer Abfolge von baugleichen Stoffaustauschböden soll in dieser Schrift als eine Querströmung, die Abfolge von solchen baugleichen Stoffaustauschböden als eine Abfolge von baugleichen Querstrom-Stoffaustauschböden und der einzelne Stoffaustauschboden innerhalb der Abfolge als Querstrom-Stoffaustauschboden bezeichnet werden.

Im einfachsten Fall ist der Querstrom-Stoffaustauschboden ein Querstrom-Siebboden. Abgesehen von dem wenigstens einen Ablaufschacht weist er Durchtrittsöffnungen für das in einer Trennkolonne aufsteigende Gas auf, für deren Ausgestaltung grundsätzlich alle beim Regensiebboden angesprochenen Ausführungsformen in Betracht kommen. Vorzugsweise weist ein Querstrom-Siebboden als Durchtrittsöffnungen ebenfalls kreisförmige Bohrungen auf, die anwendungstechnisch vorteilhaft ebenfalls einen einheitlichen Radius aufweisen. Wie bereits erwähnt, ermöglicht es der wenigstens eine Ablaufschacht, dass die in einer Trennkolonne absteigende Flüssigkeit in einer Abfolge von Querstrom-Siebböden unabhängig vom Strömungsweg des in der Abfolge aufsteigenden Dampfes (durch die Durchtrittsöffnungen hindurch) von einem höher gelegenen Querstrom-Siebboden auf den nächst tiefer gelegenen Querstrom-Siebboden absteigen kann. Auf dem tiefer gelegenen Boden fließt die Flüssigkeit im Querstrom von dem durch den wenigstens einen Ablauf des höher gelegenen Querstrom-Siebbodens gebildeten wenigstens einen Zulauf des tiefer gelegenen Bodens zu dem wenigstens einen Ablaufschacht (zu dem wenigstens einen Ablauf) des tiefer gelegenen Bodens, wobei z.B. die Höhe von wenigstens einem Ablaufwehr, über das die Flüssigkeit dem wenigstens einen Ablaufschacht zufließen kann, die gewünschte Flüssigkeitshöhe auf dem tiefer gelegenen Querstrom-Siebboden mit gewährleistet. Zusätzlich wird die Flüssigkeit durch den Staudruck des in der Trennkolonne aufsteigenden Dampfes auf dem Querstrom-Siebboden gehalten. Sinkt die Dampfbelastung eines Querstrom-Siebbodens jedoch unter einen Mindestwert, kann es zum Durchregnen der Flüssigkeit durch die Durchtrittsöffnungen kommen, was die Trennwirkung des Querstrom-Siebbodens mindert und/oder zum Trockenlaufen des Querstrom-Siebbodens führt.

Dieser Gefahr des Trockenlaufens kann dadurch entgegengewirkt werden, dass die Ablauföffnung des wenigstens einen Ablaufschachts ablaufbewehrt ist und die jeweilige Durchtrittsöffnung mit einem Hals (einem Kamin; im Fall einer kreisförmigen Durchtrittsöffnung einer Röhre) nach oben verlängert wird.

Über dem Halsende sind normalerweise Dampfumlenkhauben (Glocken, umgedrehte Tassen) angebracht (diese können im einfachsten Fall mit dem Hals (z.B. vorne und hinten) verschraubt aufsitzen und werden praktisch über den Hals gestülpt), die in die auf dem Boden aufgestaute Flüssigkeit eintauchen. Der durch die jeweilige Durchtrittsöffnung aufsteigende Dampf strömt zunächst durch deren Hals in die zugehörige Haube, in welcher er umgeleitet wird, um anschließend, im Unterschied zum Querstrom-Siebboden, parallel zur Bodenfläche aus der Haube in die auf selbiger gestaute Flüssigkeit zu strömen (eine solche "Parallelausströmung" ist bei erfindungsgemäßen Verfahren in der Regel insofern günstig, als sie in unerwünschter Weise gebildete Polymerisatpartikel "wegzublasen" und dadurch einen Selbstreinigungseffekt zu bewirken vermag). Die aus benachbarten, über den Böden vorzugsweise äquidistant verteilt angeordneten, Hauben austretenden Gasströme (Dampfströme) wirbeln die auf dem Boden gestaute Flüssigkeit auf und bilden in selbiger eine Sprudelschicht aus, in der der Stoff- und Wärmeaustausch stattfindet. Solche Querstrom-Stoffaustauschböden werden auch als Querstrom-Glockenböden bzw. Querstrom-Haubenböden bezeichnet. Da sie auch bei geringer Belastung mit aufsteigendem Gas (Dampf) gestaute Flüssigkeit aufweisen und so keine Gefahr laufen, trocken zu laufen, werden sie auch als hydraulisch abgedichtete Querstromböden bezeichnet. Im Vergleich zu Querstrom-Siebböden bedürfen sie üblicherweise höherer Investitionskosten und bedingen höhere Druckverluste des durch sie hindurch aufsteigenden Gases. Die wie beschrieben ausgeführte (ausgestaltete) Durchtrittsöffnung dieser Böden wird im Unterschied zur einfachen Siebdurchtrittsöffnung eines Siebbodens auch als Glockendurchtrittsöffnung bzw. Haubendurchtrittsöffnung bezeichnet.

Das wichtigste Bauelement des Querstrom-Glockenbodens ist die Glocke (vgl. z. B. DE 10243625 A1 und Chemie-Ing.-Techn. 45. Jahrg. 1973/Nr. 9 + 10, S. 617 bis 620). Je nach Gestalt und Anordnung der Glocken (Dampfumlenkhauben, Hauben) unterscheidet man Querstrom-Glockenböden z. B. in Querstrom-Rundglockenböden (die Querschnitte von Durchtrittsöffnung, Kamin (Hals) und Glocke (Dampfumlenkhaube) sind rund (z. B. der Zylinderglockenboden oder der Flachglockenboden), Tunnel-Querstromböden (die Querschnitte von Durchtrittsöffnung, Kamin und Glocke (Haube) sind rechteckig, die Durchtrittstellen mit ihren Glocken sind innerhalb von nebeneinander angeordneten Reihen hintereinander angeordnet, wobei die längere Rechteckkante parallel zur Querstromrichtung der Flüssigkeit ausgerichtet ist) und Querstrom-Thormann®böden (die Querschnitte von Durchtrittsöffnung, Kamin und Glocke (Haube) sind rechteckig, die Durchtrittstellen mit ihren Glocken sind innerhalb von nebeneinander angeordneten Reihen hintereinander angeordnet, wobei die längere Rechteckkante senkrecht zur Querstromrichtung der Flüssigkeit ausgerichtet ist). Querstrom-Thormannböden sind z. B. in der DE 19924532 A1 und in der DE 10243625 A1 und dem in diesen beiden Schriften gewürdigten Stand der Technik beschrieben.

Der Glockenrand kann bei Querstrom-Glockenböden sehr verschiedene Formen aufweisen (vgl. DE 10243625 A1 und Chemie-Ing. Techn. 45. Jahrg. 1973/Nr. 9 + 10, S. 617 bis 620). Abbildung 3 aus Chemie-Ing. Techn. 45 Jahr. 1973/Nr. 9 + 10, S. 618 zeigt einige Beispiele für den gezackten und den geschlitzten Rand. Die Zacken und Schlitze sind üblicherweise so geformt, dass sich der aus der Glocke heraus in die auf dem Stoffaustauschboden aufgestaute Flüssigkeit eintretende Dampf möglichst leicht in eine große Zahl von Blasen oder Dampfstrahlen auflöst. Vorgenannte Abbildung 3 sowie verschiedene Figuren der DE 10243625 A1 zeigen außerdem beispielhafte Ausführungsformen von Glockenrändern, die eine sägezahnartige Struktur aufweisen, deren Zähne zusätzlich mit Leitflügeln (Leitflächen) ausgestattet sind ("aufgebogene Schlitze"). Die Leitflügel sollen dem aus den aufgebogenen sägezahnartigen Schlitzen austretenden Gasstrom (Dampfstrom) eine tangentiale Austrittsrichtung aufzwingen (den Gasaustritt in die Flüssigkeit in eine schräge Richtung leiten) wodurch die umgebende Flüssigkeit einen gerichteten Bewegungsimpuls erhält, was im Zusammenwirken mit der Anordnung der Glocken (Dampfumlenkhauben) zu einer gerichteten Flüssigkeitsströmung auf dem Querstrom-Glockenboden führen kann, die sich der über den Stoffaustauschboden betrachtet einstellenden Querströmung überlagert (häufig werden derartige aufgebogene Schlitze auch als Treibschlitze bezeichnet). Beispielsweise fließt in einer Abfolge von Querstrom-Thormannböden die Flüssigkeit auf einem tiefer gelegenen Querstrom-Thormannboden nicht auf direktem Weg quer über den Boden, sondern auf vorstehend beschriebene Weise angetrieben mäandrierend von dem wenigstens einen Zulauf zu dem wenigstens einen Ablauf. Der Raum zwischen zwei in Querstromrichtung hintereinander angeordneten Hauben eines Querstrom-Thormannbodens bildet jeweils eine Rinne, in der die Flüssigkeit fließt. Die Detailausgestaltung eines Querstrom-Thormannbodens erfolgt darüber hinaus normalerweise so, dass die Flüssigkeit in zwei in Querstromrichtung jeweils aufeinanderfolgenden Rinnen im Gegenstrom fließt (vgl. z. B. Figur 3 der DE 10243625 A1). Das auf diese Weise resultierende Mäandrieren der Querströmung verlängert den Strömungsweg der Flüssigkeit von dem wenigstens einen Zulauf zu dem wenigstens einen Ablauf, was die Trennwirkung eines Querstrom-Thormannbodens begünstigt.

Wie bereits ausgeführt, wird bei einem Querstrom-Glockenboden das aus der Glocke austretende Gas, im Unterschied zum Querstrom-Siebboden, parallel zur Bodenfläche in die auf dem Querstrom-Glockenboden aufgestaute Flüssigkeit eingeleitet. Reibungs- und Auftriebskräfte sorgen dann dafür, dass mit zunehmendem Abstand des ausgetretenen Gasstroms vom Glockenrand immer mehr Teilströme desselben in eine Richtung senkrecht zum Querstrom-Glockenboden umgelenkt werden und schließlich aus der Flüssigkeitsschicht ausdringen. Mit zunehmender Gasbelastung einer Glocke wächst die Geschwindigkeit des aus ihr austretenden Gasstroms, was den Abstand vom Rand der Glocke ("den Wirkungsbereich der Glocke"), bis zu welchem vorstehend beschriebene Umlenkung erfolgt ist, vergrößert.

Dieser Abhängigkeit des Wirkungsbereichs einer starren Glocke von der Gasbelastung kann dadurch entgegengewirkt werden, dass die Durchtrittsöffnung eines Querstrom-Stoffaustauschbodens als Ventil (als Ventildurchtrittsöffnung) ausgestaltet (ausgeführt) wird. Die dabei resultierenden Querstrom-Stoffaustauschböden werden als Querstrom-Ventilböden bezeichnet (vgl. z. B. DD 279822 A1, DD 216633 A1 und DE 102010001228 A1).

Der Begriff Querstrom-Ventilböden subsumiert in dieser Schrift somit Querstrom-Stoffaustauschböden, die Durchtrittsöffnungen (Bodenbohrungen) mit hubbegrenzten Teller-, Ballast- oder Hebeventilen (Schwimmklappen) aufweisen, die die Größe der Dampfdurchtrittsöffnung der jeweiligen Kolonnenbelastung anpassen.

In einer einfachen Ausgestaltung werden die Durchtrittsöffnungen des Bodens zu vorgenanntem Zweck mit nach oben beweglichen Deckeln oder Tellern (Scheiben) abgedeckt. Beim Durchtritt des aufsteigenden Gases werden die Deckel (Teller, Scheiben) durch den Gasstrom in einem über die jeweilige Durchtrittsöffnung zusätzlich angebrachten (das normalerweise am Boden fest verankert ist) entsprechenden Führgerüst (Führkäfig) angehoben und erreichen schließlich eine der Gasbelastung entsprechende Hubhöhe (anstelle eines Führkäfigs kann die Scheibe auch über mit dem Boden verankerte aufwärtsbewegliche Ventilbeine verfügen, deren Aufwärtsbeweglichkeit nach oben begrenzt ist). Der durch die Durchtrittsöffnung aufsteigende Gasstrom wird an der Unterseite des angehobenen Deckels (Tellers, Scheibe) in ähnlicher Weise wie in der Glocke (bei einer Glockendurchtrittsöffnung) umgelenkt und tritt aus dem unter dem angehobenen Teller (Deckel, Scheibe) entstandenen Austrittsbereich aus und wie beim Glockenboden parallel zum Boden in die auf selbigem aufgestaute Flüssigkeit ein. Der Tellerhub steuert so die Größe des Gasaustrittsbereichs und passt sich selbständig der Kolonnenbelastung an, bis das obere Ende des Führkäfigs die maximal mögliche Hubhöhe begrenzt. Die Teller können dabei nach unten gerichtete Distanzhalter aufweisen, sodass bei niedriger Gasbelastung das Ventil nur so weit schließt, dass der durch die Distanzhalter geschaffene Raum noch eine intensive Vermischung der horizontalen Gasausströmung mit der querströmenden Flüssigkeit gestattet. Distanzhalter wirken auch einem Anhaften der Ventilscheibe am Boden entgegen. Durch geeignete Ausgestaltung der Ventilelemente eines Querstrom-Ventilbodens kann die Blasrichtung des Ventilelements eingestellt und so die Flüssigkeitszwangsführung auf dem Querstrom-Ventilboden zusätzlich beeinflusst werden (vgl. z. B. DD 216 633 A1). Das Prinzip von Querstrom-Ventilböden sowie für die Zwecke der vorliegenden Schrift verwendbare Ventilböden findet sich z. B. in Technische Fortschrittsberichte, Band 61, Grundlagen der Dimensionierung von Kolonnenböden, Seite 96 bis 138 ausgeführt. Neben den vorstehend beschriebenen beweglichen Ventilen kennt der Fachmann auch noch feststehende Ventile. Dabei handelt es sich normalerweise um scheibenförmige, oder trapezförmige, oder rechteckige Einheiten, die aus der Bodenplatte herausgestanzt werden und mit dieser über nach oben gerichtete feststehende Beine verbunden sind.

Insbesondere bei größeren Durchmessern einer Trennkolonne bildet sich auf Querstrom-Stoffaustauschböden von dem wenigstens einen Zulauf ausgehend bis zum Erreichen des Ablaufwehrs des wenigstens einen Ablaufs in natürlicher Weise ein zu beachtendes Flüssigkeitsgefälle aus (der Gradient der Stauhöhe der Flüssigkeit speist (bedingt) die Querströmung). Dies hat zur Folge, dass in Bereichen mit einer geringeren Flüssigkeitshöhe aufgrund der daraus resultierenden geringeren Widerstände der aufsteigende Dampf (das aufsteigende Gas) vergleichsweise leichter durch die Flüssigkeitsschicht hindurchtreten kann. Daraus kann schließlich eine ungleichmäßige Gasbelastung des Querstrom-Stoffaustauschbodens erwachsen (die Bereiche mit einer geringeren Flüssigkeitshöhe (einem geringeren Durchströmungswiderstand) werden bevorzugt durchströmt), die die Trennwirkung desselben beeinträchtigt. Durch die Anwendung von z. B. in ihrer Sitzhöhe einstellbaren Glocken (alternativ kann auch die Glockengröße verändert werden) bei Querstrom-Glockenböden bzw. durch Verwendung von z. B. Tellern (Deckeln) mit unterschiedlichem Gewicht bei Querstrom-Ventilböden kann diesbezüglich ausgleichend eingewirkt werden, so dass der Stoffaustauschboden über seinen Querschnitt im Wesentlichen gleichmäßig gast (dort wo die Flüssigkeitshöhe auf dem Querstrom-Stoffaustauschboden kleiner ist, wird die Sitzhöhe der Glocke anwendungstechnisch zweckmäßig entsprechend tiefer liegend bzw. das Gewicht des Hubtellers (Hubdeckels) entsprechend höher liegend gewählt; die Sitzhöhe der Glocke kann z.B. auch dadurch tiefer gelegt werden, dass die Länge des entsprechenden Kamins, an dessen Ende die Glocke gegebenenfalls verschraubt aufsitzt, gezielt verkürzt wird; alternativ oder zusätzlich kann z.B. auch die Zacken-/ Schlitzstruktur des Glockenrands variiert werden, um den erwünschten Strömungswiderstandsausgleich zu bewirken; idealer Weise erfolgt die Einstellung über den Querstrom-Stoffaustauschboden so, dass im Betrieb der Trennkolonne jede der auf einem Querstrom-Glockenboden befindliche Glocke den gleichen Strömungswiderstand für das aufsteigende Gas bedingt). Im Übrigen sind die Durchtrittstellen (die Durchtrittsöffnungen) eines Querstrom-Stoffaustauschbodens in der Regel vorteilhaft einheitlich gestaltet.

Durch einen Querstrom-Stoffaustauschboden hindurch verlaufende Öffnungen (von oben nach unten), deren Querschnittsfläche üblicherweise mehr als 200 mal kleiner als die Gesamtquerschnittsfläche aller übrigen Öffnungen des Querstromstoffaustauschbodens (den Querschnitt des wenigstens einen Ablaufschachtes nicht miteinbezogen) ist, bilden keine (trennwirksamen) Durchtrittsöffnungen für das durch den Querstrom-Stoffaustauschboden aufsteigende Gas und werden selbigen daher nicht zugerechnet.

Beispielsweise kann es sich bei solchen Öffnungen um winzige Leerlaufbohrungen handeln, über die hydraulisch abgedichtete Querstromböden beim Abschalten einer Trennkolonne leerlaufen können. Auch können solche Öffnungen Verschraubungszwecken dienen.

Abfolgen von wenigstens einen Ablaufschacht aufweisenden Stoffaustauschböden, bei denen sich der wenigstens eine Zulauf und der wenigstens eine Ablauf z. B. in der gleichen Hälfte des (kreisförmigen) Stoffaustauschbodens befinden oder bei denen sich der wenigstens eine Zulauf in Bodenmitte und der wenigstens eine Ablauf am Rand des Bodens befindet, bilden keine Abfolge von Querstrom-Stoffaustauschböden im anmeldegemäßen (erfindungsgemäßen) Sinn.

Der Wirkungsgrad von wie beschrieben ausgeführten Querstrom-Stoffaustauschböden liegt üblicherweise unterhalb desjenigen eines theoretischen Bodens (einer theoretischen Trennstufe). Als theoretischer Boden (oder theoretische Trennstufe) soll in dieser Schrift ganz generell diejenige Raumeinheit einer für ein thermisches Trennverfahren eingesetzten, trennwirksame Einbauten enthaltenden, Trennkolonne verstanden werden, die eine Stoffanreicherung entsprechend dem thermodynamischen Gleichgewicht bewirkt. D. h., der Begriff des theoretischen Bodens ist sowohl auf Trennkolonnen mit Stoffaustauschböden, als auch auf Trennkolonnen mit Packungen und/oder Füllkörpern anwendbar.

Der Stand der Technik empfiehlt den Einsatz von Abfolgen von wenigstens zwei baugleichen (identisch ausgeführten) Querstrom- Stoffaustauschböden u. a. in trennwirksame Einbauten enthaltenden Trennkolonnen, die zur Durchführung thermischer Trennverfahren zwischen wenigstens einem in der Trennkolonne aufsteigenden Gasstrom und wenigstens einem in der Trennkolonne absteigenden Flüssigkeitsstrom zur Anwendung kommen, und wobei wenigstens einer der Ströme wenigstens ein (Meth)acrylmonomeres enthält. Beispielsweise empfehlen die Schriften DE 19924532 A1, DE 10243625 A1 und WO 2008/090190 A1 die Mitverwendung einer Abfolge baugleicher hydraulisch abgedichteter Querstrom-Stoffaustauschböden in einer Trennkolonne zur Durchführung eines Verfahrens der fraktionierenden Kondensation eines Acrylsäure enthaltenden Produktgasgemischs einer heterogen katalysierten Gasphasen-Partialoxidation von C₃-Vorläufern der Acrylsäure mit molekularem Sauerstoff, die von unten nach oben zunächst Dual-Flow-Böden und im Anschluss daran hydraulisch abgedichtete Querstrom-Stoffaustauschböden enthält.

Charakteristisch für die Abfolgen der im Stand der Technik empfohlenen Querstrom-Stoffaustauschböden ist, dass der jeweils untere von zwei in der Abfolge aufeinanderfolgenden Querstrom-Stoffaustauschböden in Richtung der Querströmung von seinem wenigstens einen Zulauf zu seinem wenigstens einen Ablaufschacht nur im Bereich zwischen dem wenigstens einen Zulauf und dem wenigstens einen Ablaufschacht (der wenigstens einen Ablauföffnung) Durchtrittsöffnungen aufweist (vgl. z. B. die Figuren 3 und 4 der DE 10243625 A1, die Figur 1 der DD 279822 A1, die Figur 1 der DD 216633 A1 und die Abbildung 1 aus Chemie-Ing.-Techn. 45. Jahrgang, 1973/Nr. 9 + 10, Seite 617 bis 620).

Die Erfindung betrifft insbesondere Kolonnen, bei denen die vorgenannten Böden eingesetzt werden.

Eine problematische Eigenschaft von (Meth)acrylmonomeren ist deren Neigung zu unerwünschter Polymerisation, die sich, insbesondere in flüssiger Phase befindlich, auch durch den Zusatz von Polymerisationsinhibitoren nicht vollständig unterdrücken lässt.

Nachteilig an bekannten Trennkolonnen ist, dass es bei kontinuierlicher Durchführung des thermischen Trennverfahrens während längerer Betriebsdauern bei den Stoffaustauschböden vergleichsweise häufig zur Ausbildung von unerwünschtem Polymerisat kommt. Dies ist insbesondere deshalb von Nachteil, weil der Betreiber des thermischen Trennverfahrens aufgrund der unerwünschten Polymerisatbildung das thermische Trennverfahren immer wieder unterbrechen muss, um das gebildete Polymerisat zu entfernen. Selbiges kann nämlich die Durchtrittsöffnungen des Stoffaustauschbodens teilweise oder vollständig verschließen. Außerdem ist die radikalische Polymerisation von (Meth)acrylmonomere normalerweise ausgeprägt exotherm, d. h. unter starker Wärmeentwicklung. Es besteht die Gefahr, dass die Polymerisation so heftig verläuft, dass die Trennkolonne, welche das Polymerisationsgemisch enthält, explodiert.

Um bestimmte Revisionsarbeiten in der Kolonne vornehmen zu können oder um den Kolonnenhohlraum zu reinigen, sind üblicherweise Revisionsöffnungen im Kolonnenkörper vorgesehen. Eine solche Revisionsöffnung ist z.B. bei einem Stutzen gebildet, der in dem Kolonnenkörper angeordnet ist. Der Durchmesser der Revisionsöffnung ist an die beabsichtigte Funktion der Revisionsöffnung angepasst. Es kann sich um ein so genanntes Handloch handeln, durch welches eine Person ihre Hand beispielsweise zusammen mit einer Reinigungsvorrichtung einführen kann. Des Weiteren kann die Revisionsöffnung als Mannloch ausgeführt sein, bei dem der Durchmesser der Öffnung so groß ist, dass im Nicht-Betrieb ein Arbeiter in den Hohlraum der Kolonnen einsteigen kann, um dort Revisions- und Reinigungsarbeiten vorzunehmen. Über die Revisionsöffnung kann beispielsweise auch beim Betrieb der Kolonne in unerwünschter Weise gebildetes Polymerisat der Acrylsäure entfernt werden.

Die in dem Kolonnenhohlraum montierten Böden sind üblicherweise so angeordnet, dass sich die Revisionsöffnung zwischen zwei Böden befindet. Wenn die Revisionsöffnung allerdings als Mannloch ausgebildet ist, hat dies den Nachteil, dass der Abstand zwischen den Böden unerwünscht groß wird. Wenn im Bereich der Revisionsöffnung keine trennwirksamen Einbauten vorgesehen sind, kann sich in diesem Bereich unerwünschtes Polymerisat bilden.

Zur Lösung dieses Problems wurde in der WO 2013/139590 A1 vorgeschlagen, auch im Mannlochbereich einer Kondensationskolonne trennwirksame Einbauten anzubringen und auf diese Weise den Abstand zum Übergangsboden zu verringern. Das Problem, dass sich unerwünschtes Polymerisat im Bereich der Revisionsöffnung bildet, bleibt jedoch bestehen.

Aus der US 6 585 862 B1 ist eine Kolonne bekannt, bei welcher ein Stutzen für eine verschließbare Revisionsöffnung ausgebildet ist. Mittels einer Sprüheinrichtung kann Flüssigkeit gegen die in den Kolonnenhohlraum gerichtete Oberfläche des Stutzens gesprüht werden. Solche Sprüheinrichtungen sind auch in den folgenden Druckschriften beschrieben: US 6 409 886 B1, US 2011/0240458 A1 und EP 1 561 521 A2.

Der vorliegenden Erfindung lag daher die Aufgabe zugrunde, eine Kolonne und ein thermisches Trennverfahren der eingangs genannten Art bereitzustellen, bei denen eine Polymerisation des in der Trennkolonne befindlichen Stoffs verhindert oder zumindest verringert werden kann.

Erfindungsgemäß wird diese Aufgabe durch eine Kolonne mit den Merkmalen des Anspruchs 1 sowie ein thermisches Trennverfahren mit den Merkmalen des Anspruchs 11 gelöst. Vorteilhafte Ausgestaltungen und Weiterbildungen ergeben sich aus den abhängigen Ansprüchen.

Demgemäß wurde eine Kolonne zur thermischen Behandlung von fluiden Gemischen gefunden, die einen zylindrischen, vertikal ausgerichteten Kolonnenkörper, der einen Kolonnenhohlraum bildet, mehrere im Kolonnenhohlraum montierte Böden, die vertikal beabstandet voneinander angeordnet sind, zumindest einen Stutzen, der in dem Kolonnenkörper angeordnet ist und der sich von dem Kolonnenkörper weg erstreckt, und eine verschließbare Revisionsöffnung aufweist, die bei dem Stutzen gebildet ist, wobei in dem Kolonnenkörper eine Sprüheinrichtung angeordnet ist, mit der Flüssigkeit zumindest gegen die in den Kolonnenhohlraum gerichtete Oberfläche des Stutzens, d. h. die Innenoberfläche des Stutzens, sprühbar ist.

Die räumlichen Begriffe "oben", "unten", "horizontal" und "vertikal", beziehen sich, soweit nichts anderes ausdrücklich erwähnt ist, auf die Orientierung der Kolonne während des Betriebs.

Es wurde gefunden, dass sich unerwünschtes Polymerisat insbesondere in sogenannten Totzonen in der Kolonne bildet. Bei solchen Totzonen ist die Verweilzeit des Fluids in der Kolonne besonders lang. Eine solche lange Verweilzeit begünstigt die Polymerisation. Es hat sich herausgestellt, dass sich Totzonen insbesondere im Bereich der Revisionsöffnung bilden können. Erfindungsgemäß kann diese Polymerisatbildung dadurch verhindert werden, dass während des Betriebs der Kolonne die Innenoberfläche des Stutzens mittels der Sprüheinrichtung mit Flüssigkeit besprüht wird, so dass verhindert wird, dass Flüssigkeit länger bei der Innenoberfläche des Stutzens verweilt.

Erfindungsgemäß weist die Sprüheinrichtung eine Sprühdüse, eine Zuleitung und eine Sprühflüssigkeits-Zuführeinrichtung auf. Die Sprühflüssigkeits-Zuführeinrichtung ist ausgebildet, Sprühflüssigkeit dem Kolonnenhohlraum zu entnehmen, die entnommene Sprühflüssigkeit über die Zuleitung der Sprühdüse zuzuführen und mittels der Sprühdüse zumindest gegen die Innenoberfläche des Stutzens zu sprühen. Die Sprühflüssigkeit wird dabei insbesondere oberhalb eines im Kolonnenhohlraum montierten Bodens und insbesondere nicht dem Kolonnensumpf entnommen. Durch die Verwendung der im Kolonnenhohlraum befindlichen Flüssigkeit als Sprühflüssigkeit ergibt sich der Vorteil, dass die Sprühflüssigkeit im Wesentlichen dieselbe Zusammensetzung hat wie die bei der Innenoberfläche des Stutzens weggespülte Flüssigkeit.

Die Sprühflüssigkeits-Zuführeinrichtung weist erfindungsgemäß eine Einlauföffnung auf, die unmittelbar oberhalb eines Bodens angeordnet ist, der benachbart zu dem Stutzen ist. Bevorzugt ist die Einlauföffnung unmittelbar oberhalb des Bodens angeordnet, der unmittelbar unterhalb des Stutzens angeordnet ist. In diesem Fall wird die Sprühflüssigkeit dem
Kolonnenhohlraum in einem Bereich entnommen, der benachbart zu der Höhe des Stutzens ist, insbesondere direkt unter dem Stutzen. Dies hat den Vorteil, dass die Sprühflüssigkeit auf derselben Stufe der Kolonne entnommen wird, so dass die Sprühflüssigkeit und die bei der Innenoberfläche des Stutzens abzuspülende Flüssigkeit die gleiche Zusammensetzung haben. Dies wirkt sich positiv auf die Trennwirkung eines Verfahrens aus, das mit der erfindungsgemäßen Kolonne durchgeführt wird.

Gemäß einer weiteren Ausgestaltung der erfindungsgemäßen Kolonne bildet der Kolonnenkörper eine vertikale Innenoberfläche. Bei einem vertikalen Schnitt der Kolonne schließt die in den Kolonnenhohlraum gerichtete Linie der unteren Schnittlinie des Stutzens, die ein Teil der Oberfläche des Stutzens ist, oder eine Tangente an dieser Linie der unteren Schnittlinie des Stutzens zumindest abschnittsweise mit der vertikalen Innenlinie des Kolonnenkörpers, die sich von dem Stutzen nach unten erstreckt und die ein Teil der Innenoberfläche des Kolonnenkörpers ist, einen Winkel in einem Bereich von 210° bis 267° ein. In einer vorteilhaften Ausgestaltung ist dieser Winkel in einem Bereich von 225° bis 267° und bevorzugt in einem Bereich von 255° bis 267°.

Es hat sich herausgestellt, dass sich Totzonen insbesondere im unteren Teil des Stutzens bilden können. Üblicherweise erstreckt sich die untere Wand des Stutzens horizontal von dem Kolonnenkörper weg. Auf dieser horizontalen Fläche kann sich jedoch Flüssigkeit ansammeln, die länger in der Kolonne verweilt. Wenn ein polymerisierbarer Stoff in der Kolonne behandelt wird, kommt es somit auf dieser horizontalen Fläche des Stutzens der Revisionsöffnung zu einer unerwünschten Polymerisatbildung. Erfindungsgemäß kann diese Polymerisatbildung dadurch verhindert werden, dass der untere Teil des Stutzens so geneigt ist, dass Flüssigkeit, welche sich an der in den Kolonnenhohlraum gerichteten Oberfläche des Stutzens niederschlägt, zurück in den Kolonnenhohlraum abläuft. Der Neigungswinkel sollte zumindest 3° sein, in diesem Fall ist der Winkel der in den Kolonnenhohlraum gerichteten Linie der unteren Schnittlinie des Stutzens mit der vertikalen Innenlinie des Kolonnenkörpers, die sich von dem Stutzen nach unten erstreckt, 267°. Die Neigung ist bevorzugt noch größer, wobei zu große Neigungen zu größer werdenden Öffnungen im Kolonnenkörper für den Stutzen führen. Die Wahl des Winkels stellt somit einen Kompromiss zwischen einer geeigneten Neigung der unteren Fläche des Stutzens zur Horizontalen einerseits und einem geeigneten Durchmesser des Stutzens andererseits dar.

Gemäß einer weiteren Ausgestaltung der erfindungsgemäßen Kolonne schließt bei einem vertikalen Querschnitt der Kolonne zumindest 50% der in den Kolonnenhohlraum gerichteten Linie der unteren Schnittlinie des Stutzens oder die Tangente an zumindest 50% dieser Linie der unteren Schnittlinie des Stutzens mit der vertikalen Innenlinie des Kolonnenkörpers, die sich von dem Stutzen nach unten erstreckt, einen Winkel in einem Bereich von 210° bis 267° ein, bevorzugt einen Winkel in einem Bereich von 225° bis 267° und besonders bevorzugt einen Winkel in einem Bereich von 255° bis 267° ein. In diesem Fall kann abschnittsweise die Neigung, d. h. der Winkel gegenüber der Horizontalen, der unteren Oberfläche des Stutzens auch geringer sein, d. h. der vorstehend genannte Winkel größer als der angegebene Winkel sein. Bevorzugt fallen jedoch 70%, weiter bevorzugt 90% und insbesondere 100% der unteren Schnittlinie des Stutzens in den genannten Winkelbereich.

Der Stutzen der Kolonne weist eine obere Hälfte und eine untere Hälfte auf. Bei der erfindungsgemäßen Kolonne schließt insbesondere bei der unteren Hälfte die in den Kolonnenhohlraum gerichtete Oberfläche des Stutzens oder die Tangente an der Oberfläche des Stutzens mit der vertikalen Innenoberfläche des Kolonnenkörpers, die sich von dem Stutzen nach unten erstreckt, einen Winkel in einem Bereich von 210° bis 267° ein, bevorzugt einen Winkel in einem Bereich von 225° bis 267° und besonders bevorzugt einen Winkel in einem Bereich von 255° bis 267° ein. Das unerwünschte Polymerisat bildet sich nämlich insbesondere bei den Oberflächen dieser unteren Hälfte des Stutzens. Durch die genannte Wahl des Winkels zu der vertikalen Innenoberfläche des Kolonnenkörpers wird verhindert, dass Flüssigkeit bei den Oberflächen der unteren Hälfte des Stutzens verbleibt und Polymerisat bildet.

Anwendungstechnisch vorteilhaft ist der Stutzen jedoch um eine horizontale Achse rotationssymmetrisch ausgebildet. In diesem Fall schließt die gesamte in den Kolonnenhohlraum gerichtete Oberfläche des Stutzens oder die Tangente auf der Oberfläche des Stutzens mit der vertikalen Innenoberfläche des Kolonnenkörpers einen Winkel in einem Bereich von 210° bis 267° ein, bevorzugt einen Winkel in einem Bereich von 225° bis 267° und besonders bevorzugt einen Winkel in einem Bereich von 255° bis 267° ein.

Der Stutzen kann beispielsweise kegelstumpfförmig ausgebildet sein. Die in den Kolonnenhohlraum gerichtete Oberfläche des Stutzens schließt dann mit der vertikalen Innenoberfläche des Kolonnenkörpers einen Winkel ein, der in dem vorstehend genannten Bereich liegt. Bei einem kegelstumpfförmigen Stutzen kann Flüssigkeit, sich bei der Oberfläche des Stutzens niederschlägt, besonders gut zurück in den Kolonnenhohlraum ablaufen.

Gemäß einer anderen Ausgestaltung der erfindungsgemäßen Kolonne erstreckt sich der Stutzen horizontal von dem vertikal ausgerichteten Kolonnenkörper weg. In diesem Fall wird die Polymerisatbildung auf dem horizontal ausgerichteten Bereich des Stutzens mittels der Sprüheinrichtung verhindert.

Der Stutzen ist insbesondere in vertikaler Richtung zwischen zwei in dem Kolonnenhohlraum montierten Böden angeordnet. Diese beiden Böden müssen nicht notwendigerweise benachbart seien. Es können sich auch weitere Böden zwischen diesen beiden Böden im Bereich des Stutzens befinden.

Gemäß einer weiteren Ausgestaltung der erfindungsgemäßen Kolonne ist die Revisionsöffnung eine Mannlochöffnung, die bei dem Stutzen gebildet ist und die mit einem Deckel verschließbar ist. Wenn in diesem Fall zumindest einer der Böden im Bereich der Mannlochöffnung montiert ist, ist vorteilhafterweise im Bereich des Stutzens zwischen dem einen Boden und dem geschlossenen Deckel eine Platte angeordnet. Durch diese Platte kann vorteilhafterweise verhindert werden, dass aufsteigendes Gas oder absteigende Flüssigkeit durch eine horizontale Öffnung im Bereich der Mannlochöffnung an dem Boden, der im Bereich der Mannlochöffnung montiert ist, vorbeiströmt.

Bevorzugt ist der gesamte Querschnitt des Kolonnenkörpers im Bereich des Stutzens von dem einen Boden im Bereich der Mannlochöffnung und der Platte im Wesentlichen ausgefüllt. Nur bei den Fugen verbleiben ggf. Öffnungen. Wenn es sich bei dem Boden im Bereich der Mannlochöffnung um einen Stoffaustauschboden mit Öffnungen handelt, ist die Platte bevorzugt als Stoffaustauschplatte ausgebildet. Diese Stoffaustauschplatte weist insbesondere auch Öffnungen auf, durch welche Gas aufsteigen und Flüssigkeit absteigen kann, wodurch ein Stoffaustausch bewirkt wird.

Der eine Boden im Bereich der Mannlochöffnung und die Platte sind insbesondere im Wesentlichen horizontal ausgerichtet. Der Deckel kann schwenkbar an dem Stutzen befestigt sein. Ferner kann die Platte an dem Deckel befestigt sein, so dass sie entfernt wird, wenn der Deckel von dem Stutzen abgenommen oder weggeschwenkt wird.

Die bei dem Stutzen gebildete Revisionsöffnung besitzt insbesondere einen kreisförmigen Querschnitt. Es sind jedoch andere runde, ovale oder seltener auch rechteckige Querschnitte möglich. Die lichte Weite der Revisionsöffnung ist in einem Bereich von 100 mm bis 800 mm. Wenn die Revisionsöffnung als Mannlochöffnung ausgebildet ist, ist die lichte Weite insbesondere in einem Bereich von 400 mm bis 800 mm. Nur wenn angedacht ist, große Werkzeuge oder sonstige große Teile mit durch die Mannlochöffnung zu nehmen, kann diese Öffnung noch größer ausgeführt werden. Wenn die Revisionsöffnung als Handlochöffnung ausgebildet ist, ist die lichte Weite geringer, insbesondere in einem Bereich von 100 mm bis 300 mm.

Bei dem Boden, der in der erfindungsgemäßen Kolonne eingesetzt wird, handelt es sich insbesondere um einen Dual-Flow-Boden. Bei Dual-Flow-Böden ist das Risiko der Polymerisation bei einem Einsatz eines fluiden Gemisches, welches (Meth)acrylmonomere enthält, besonders hoch. Durch die erfindungsgemäße Kolonne kann in diesem Fall besonders wirkungsvoll die Bildung von Polymerisat und damit das Explosionsrisiko verringert werden.

Bei den Böden, die in der Kolonne montiert sind, kann es sich jedoch auch um andere Böden handeln, wie sie einleitend beschrieben wurden. Zwischen den Böden können weitere trennwirksame Einbauten angeordnet sein. Durch die trennwirksamen Einbauten wird die Stofftrennung in einer Kolonne, die als Trennkolonne eingesetzt wird, verbessert.

Diese weiteren Einbauten können beispielsweise in Form von Packungen, insbesondere strukturierten bzw. geordneten Packungen, und/oder Schüttungen vorgesehen sein. Unter den Schüttungen sind solche mit Ringen, Wendeln, Sattelkörpern, Raschig-, Intos- oder Pall-Ringen, Berl- oder Intalox-Sätteln, Top-Pak etc. bevorzugt. Für erfindungsgemäß zu verwendende Extraktionskolonnen besonders geeignete Packungen sind z. B. Packungen der Julius Montz GmbH in D-40705 Hilden, wie z. B. die Packung Montz-Pak B1-350. Vorzugsweise verwendet man gelochte strukturierte Packungen aus Edelstahlblechen. Packungskolonnen mit geordneten Packungen sind dem Fachmann an sich bekannt und z. B. in Chem.-Ing.Tech. 58 (1986) Nr. 1, S. 19-31 sowie in der Technischen Rundschau Sulzer 2/1979, S. 49 ff. der Gebrüder Sulzer Aktiengesellschaft in CH-Winterthur beschrieben.

Die erfindungsgemäße Kolonne kann insbesondere als Trennkolonne eingesetzt werden. Die Trennkolonne weist eine Abfolge von Böden auf. Der lichte Abstand zwischen zwei innerhalb der erfindungsgemäßen Kolonne unmittelbar aufeinanderfolgenden Böden beträgt insbesondere nicht mehr als 700 mm, vorzugsweise nicht mehr als 600 mm bzw. nicht mehr als 500 mm. Anwendungstechnisch zweckmäßig ist der lichte Abstand innerhalb der Bodenabfolge 300 bis 500 mm. Im Regelfall sollte der Bodenabstand 250 mm nicht unterschreiten.

Die Höhe des Kolonnenkörpers ist beispielsweise größer als 5 m, insbesondere größer als 10 m. Es ist jedoch auch möglich, dass die Höhe des Kolonnenkörpers 30 m oder 40 m übersteigt.

Die Erfindung betrifft des Weiteren ein thermisches Trennverfahren zwischen wenigstens einem in einer Kolonne, wie sie vorstehend beschrieben wurde, aufsteigenden Gas und wenigstens einer in der Kolonne absteigenden Flüssigkeit. Dabei enthält das aufsteigende Gas und/oder die absteigenden Flüssigkeit insbesondere (Meth)acrylmonomere.

Das erfindungsgemäße thermische Trennverfahren kann z.B. ein Verfahren der fraktionierenden Kondensation zur Abtrennung von Acrylsäure aus einem Acrylsäure enthaltenden Produktgasgemisch einer heterogen katalysierten Gasphasenpartialoxidation einer C3-Vorläuferverbindung (insbesondere Propen und/oder Propan) der Acrylsäure mit molekularem Sauerstoff zu Acrylsäure sein.

Die Trennkolonne (Kondensationskolonne) kann wie in den Schriften DE 10243625 A1 bzw. WO 2008/090190 A1 beschrieben ausgeführt sein.

Im Folgenden werden Ausführungsbeispiele der erfindungsgemäßen Kolonne und Ausführungsbeispiele des erfindungsgemäßen Verfahrens mit Bezug zu den Zeichnungen erläutert.
- Figur 1: zeigt eine schematische Ansicht einer Kolonne gemäß einem Ausführungsbeispiel der Erfindung,
- Figur 2: zeigt eine Detailansicht eines vertikalen Querschnitts der in Figur 1 gezeigten Kolonne im Bereich einer Revisionsöffnung,
- Figur 3: zeigt eine Detailansicht eines vertikalen Querschnitts eines weiteren Ausführungsbeispiels der erfindungsgemäßen Kolonne,
- Figur 4: zeigt eine Detailansicht eines vertikalen Querschnitts eines noch weiteren Ausführungsbeispiels der erfindungsgemäßen Kolonne und
- Figur 5: zeigt einen horizontalen Querschnitt der in Figur 4 gezeigten Kolonne im Bereich der Revisionsöffnung.

Das im Folgenden beschriebene Ausführungsbeispiel betrifft eine Trennkolonne 1, wie sie z. B. bei einem Verfahren der fraktionierenden Kondensation zur Abtrennung von Acrylsäure aus einem Acrylsäure enthaltenden Produktgasgemisch einer heterogen katalysierten Gasphasenpartialoxidation einer C3-Vorläuferverbindung (insbesondere Propen und/oder Propan) der Acrylsäure mit molekularem Sauerstoff zu Acrylsäure eingesetzt wird.

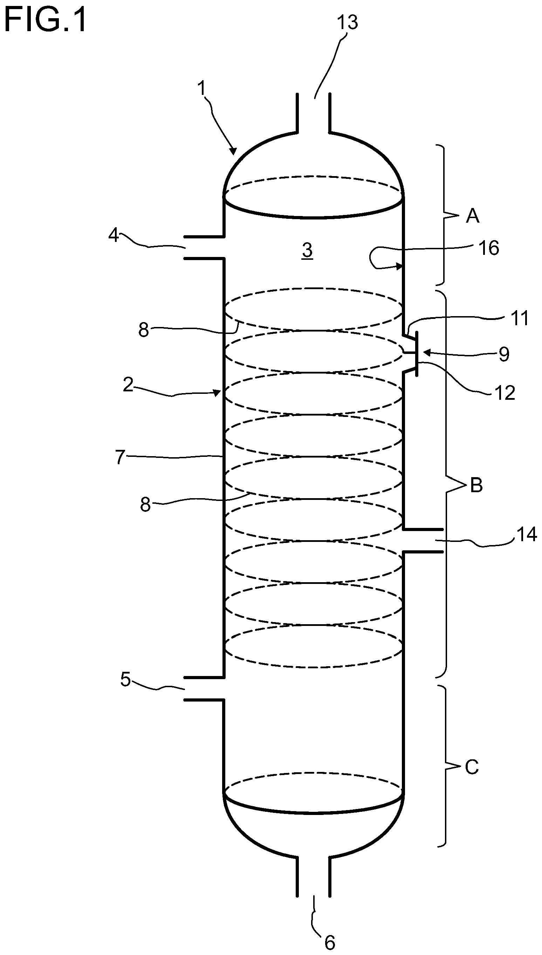

In Fig. 1 ist die an sich bekannte Trennkolonne 1 schematisch dargestellt. Sie umfasst einen zylindrischen Kolonnenkörper 2, dessen Achse vertikal ausgerichtet ist. Bei dem Kolonnenkörper 2 handelt es sich im Wesentlichen um einen Hohlzylinder. Das heißt, der Mantel 7 des Kolonnenkörpers 2 bildet einen Kolonnenhohlraum 3. Der Kolonnenkörper 2 ist aus Edelstahl gefertigt. Nach außen ist die Trennkolonne 1 normalerweise in herkömmlicher Weise thermisch isoliert. Die Höhe der Trennkolonne 1 ist 40 m. Der Innendurchmesser des Mantels 7 des Kolonnenkörpers 2 beträgt durchgehend 7,4 m.

In vertikaler Richtung ist die Trennkolonne 1 in drei Bereiche unterteilt: Der obere Bereich A wird als Kolonnenkopf bezeichnet. Beim Kolonnenkopf ist ein Zulauf 4 vorgesehen, über welchen eine Flüssigkeit in den Kolonnenhohlraum 3 eingeleitet werden kann. Ferner ist oben eine Abgasleitung 13 zur Entnahme des gasförmigen Gemisches ausgebildet.

Unterhalb des Kolonnenkopfes ist ein Bereich B gebildet. In diesem Bereich wird die fraktionierende Kondensation durchgeführt. Im Bereich B ist eine Entnahmeleitung 14 angeordnet, über welche Rohacrylsäure entnommen wird.

Unterhalb des Bereichs B ist der Kolonnensumpf im Bereich C gebildet. Beim Kolonnensumpf befindet sich eine Zuleitung 5 zum Einleiten des Produktgasgemisches in den Kolonnenhohlraum 3. Ferner befindet sich im Kolonnensumpf ein Ablauf 6 für die Sumpfflüssigkeit.

Im Bereich B sind im Kolonnenhohlraum 3 mehrere Böden 8 befestigt. Die Böden 8 der Kolonne 1 sind horizontal ausgerichtet und vertikal beabstandet voneinander in dem Kolonnenhohlraum 3 montiert. Dabei werden bei den Böden 8 nach unten ausgerichtete horizontale Flächen gebildet. Die Böden 8 dienen als trennwirksame Einbauten, welche die Stofftrennung in der Trennkolonne 1 verbessern. Bei den Böden 8 handelt es sich um Dual-Flow-Böden. Es können aber auch andere der einleitend genannten Böden verwendet werden.

Um im Nicht-Betrieb der Kolonne 1 Revisions- und Reinigungsarbeiten vornehmen zu können, ist im Kolonnenkörper 2 zumindest eine Revisionsöffnung 9 ausgebildet. Hierfür weist der Mantel 7 bzw. der Kolonnenkörper 2 eine Öffnung auf. Der Querschnitt der Öffnung ist kreisrund. Je nach Bedarf können jedoch auch andere Querschnittsformen verwendet werden. Am Rand dieser Öffnung ist flüssigkeits- und gasdicht ein kegelstumpfförmiger Stutzen 11 befestigt. Die Symmetrieachse des Stutzens 11 ist horizontal ausgerichtet, so dass sich der Stutzen 11 von dem Kolonnenkörper 2 weg erstreckt. Das von dem Kolonnenkörper 2 abgewandte Ende des Stutzens 11 bildet die Revisionsöffnung 9. An diesem Ende ist auch ein Deckel 12 vorgesehen. Der Deckel 12 ist schwenkbar an dem Stutzen 11 befestigt. Im geschlossenen Zustand verschließt der Deckel 12 die Revisionsöffnung 9 flüssigkeits- und gasdicht. Im geöffneten, aufgeschwenkten Zustand des Deckels 12 ist der Kolonnenhohlraum 3 über die Revisionsöffnung 9 von außen zugänglich.

In Figur 1 ist nur ein Stutzen 11 gezeigt. Üblicherweise umfasst der Kolonnenkörper 2 mehrere in vertikaler Richtung beabstandete Stutzen 11 mit den dazugehörigen Revisionsöffnungen 9.

Der Durchmesser der Revisionsöffnung 9 richtet sich nach dem Zweck der Revisionsöffnung 9. Im hier beschriebenen Ausführungsbeispiel ist die Revisionsöffnung 9 als Mannlochöffnung ausgebildet. Der Durchmesser dieser Mannlochöffnung ist in einem Bereich von 400 mm bis 800 mm.

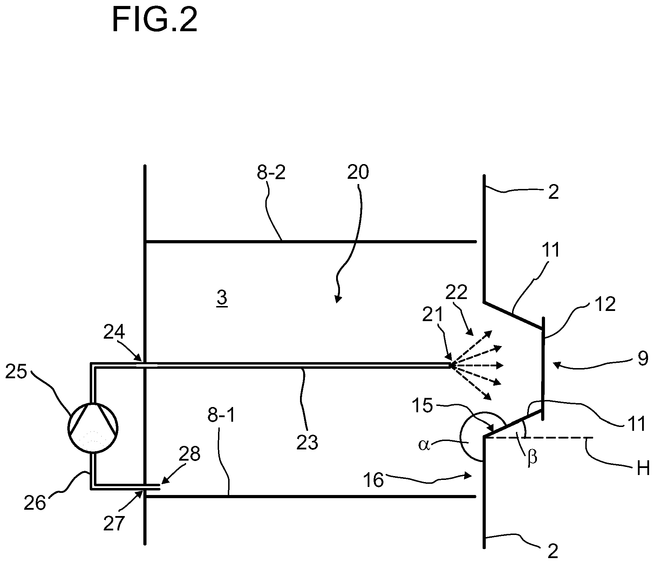

In Figur 2 ist die Ausgestaltung der Revisionsöffnung 9 im Detail gezeigt. Der Kolonnenkörper 2 besitzt eine in den Kolonnenhohlraum 3 gerichtete vertikale Innenoberfläche 16. Ferner besitzt auch der Stutzen 11 eine in den Kolonnenhohlraum 3 gerichtete Oberfläche 15. Dies ist die Innenfläche des Stutzens 11.

Unterhalb und oberhalb der Revisionsöffnung 9 ist ein Stoffaustauschboden 8-1 und 8-2 angeordnet. Da es sich bei der Revisionsöffnung 9 um eine Mannlochöffnung handelt, ist der Abstand zwischen diesen Stoffaustauschböden 8-1 und 8-2 relativ groß, beispielsweise 1000 mm. Dieser relativ große Abstand zwischen den beiden Stoffaustauschböden 8-1 und 8-2 kann zu einer unerwünschten Polymerisatbildung führen. Um eine Polymerisation im Bereich zwischen den Stoffaustauschböden 8-1 und 8-2, insbesondere bei dem Stutzen 11 zu verhindern, ist in dem Kolonnenkörper 2 eine Sprüheinrichtung 20 angeordnet. Mittels der Sprüheinrichtung 20 ist eine Flüssigkeit 22 zumindest gegen die in den Kolonnenhohlraum 3 gerichtete Oberfläche 15 des Stutzens 11 sprühbar. Hierfür weist die Sprüheinrichtung 20 eine Sprühdüse 21 auf, welcher über eine Zuleitung 23 Flüssigkeit zugeführt wird. Die Zuleitung 23 tritt durch eine gas- und flüssigkeitsdichte Durchführung 24 durch den Kolonnenkörper 2 durch. Außerhalb des Kolonnenkörpers 2 ist eine Pumpe 25 angeordnet, welche mit der Zuleitung 23 verbunden ist. Auf der anderen Seite ist die Pumpe 25 mit einer Leitung 26 verbunden, welche über eine weitere gas- und flüssigkeitsdichte Durchführung 27 wieder in den Kolonnenhohlraum eintritt. Die Leitung 26 weist eine Einlauföffnung 28 auf, welche unmittelbar oberhalb des Stoffaustauschbodens 8-1 angeordnet ist. Der Stoffaustauschboden 8-1 ist dabei benachbart zu der Revisionsöffnung 9 bzw. dem Stutzen 11.

Im hier gezeigten Ausführungsbeispiel ist dieser Stoffaustauschboden 8-1 unmittelbar unterhalb der Revisionsöffnung 9. Mittels der Sprüheinrichtung 20 wird Flüssigkeit, welche sich auf dem Stoffaustauschboden 8-1 gesammelt hat, entnommen und von der Sprühdüse 21 gegen die Oberfläche 15 des Stutzens 11 sowie die Innenfläche des Deckels 12 besprüht. Hierdurch wird verhindert, dass sich Flüssigkeit in diesem Bereich ansammelt und polymerisiert.

Bei dem in Figur 2 gezeigten vertikalen Querschnitt der Kolonne 1 schließt die Oberfläche 15 der unteren Schnittlinie des Stutzens 11, die in Figur 2 wegen der Schnittdarstellung als Linie gezeigt ist, mit der vertikalen Innenoberfläche 16 des Kolonnenkörpers 2, die sich von dem Stutzen 11 nach unten erstreckt und die in Figur 2 wegen der Schnittdarstellung auch als Linie gezeigt ist, den Winkel α ein. Am Scheitel des Winkels sind die vertikale Innenoberfläche 16 des Kolonnenkörpers 2 und die untere Schnittlinie des Stutzens 11 somit verbunden. Entsprechend schließt diese Oberfläche 15 des Stutzens 11 mit der Horizontalen H den Winkel β ein, wobei die Summe der Winkel α und β 270° ist.

Bei der in Figur 2 gezeigten Ausgestaltung ist der Winkel β größer als 0, d. h. die Oberfläche 15 ist bei der unteren Schnittlinie des vertikalen Querschnitts der Kolonne 1 nicht horizontal ausgerichtet, sondern geneigt. Der Neigungswinkel ist im vorliegenden Ausführungsbeispiel 3°, wobei die Zeichnungen die Winkel zur besseren Veranschaulichung nicht wirklichkeitsgetreu wiedergeben. Der Winkel α ist in diesem Fall somit 267°.

Es wird darauf hingewiesen, dass der Winkel α auch kleiner sein kann, so dass sich eine stärkere Neigung der Oberfläche 15 ergibt. Der Winkel α ist z. B. in einem Bereich von 210° bis 267°, insbesondere in einem Bereich von 225° bis 267° und bevorzugt in einem Bereich von 255° bis 267°.

Die Neigung der Oberfläche 15 der unteren Schnittlinie des Stutzens 11 bei einem vertikalen Querschnitt der Kolonne 1 bewirkt, dass Flüssigkeit auf dieser Oberfläche 15 nach unten abläuft und insbesondere nicht auf dieser Oberfläche 15 verbleibt. Auf diese Weise kann die Polymerisation von Flüssigkeit, die (Meth)acrylmonomere enthält, verhindert werden.

Die Neigung von zumindest 3° ist insbesondere im unteren Bereich des Stutzens 11 vorteilhaft, damit Flüssigkeit ablaufen kann. Insbesondere weist die untere Hälfte des Stutzens 11 diesen Winkel zur Innenoberfläche 16 des Kolonnenkörpers 2 auf. Aus fertigungstechnischen Gründen ist der Stutzen 11 jedoch bevorzugt rotationssymmetrisch ausgebildet, so dass der Winkel zwischen der in den Kolonnenhohlraum 3 gerichteten Oberfläche des Stutzens 11 mit der Innenoberfläche 16 des Kolonnenkörpers 2 über den gesamten Umfang des Stutzens 11 gleich ist. Im Querschnitt ist die Innenlinie, die Teil der Innenoberfläche 15 des Stutzens 11 ist, eine gerade Linie. Bei anderen Ausführungsbeispielen kann diese Linie jedoch auch gebogen sein. In diesem Fall wird für den Winkel α bzw. den Winkel β die Tangente an der Oberfläche 15 der unteren Schnittlinie des Stutzens 11 mit der vertikalen Innenoberfläche 16 des Kolonnenkörpers 2 betrachtet. Bei einer gebogenen Linie ändert sich die Ausrichtung dieser Tangente. Der vorstehend angegebene Winkel α ist in diesem Fall zumindest bei 50%, bevorzugt über einen größeren Bereich, wie z. B. 70% oder 90% in dem angegebenen Winkelbereich. Der Winkel α ist insbesondere in keinem Bereich 270° oder größer.

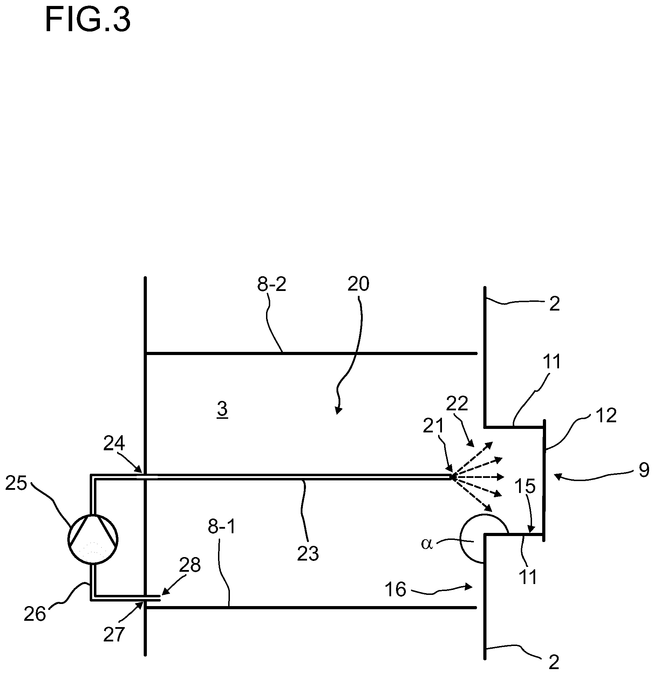

Bei dem in Figur 3 gezeigten Ausführungsbeispiel der erfindungsgemäßen Kolonne 1 ist der Winkel α 270°, d.h. die Oberfläche 15 ist in diesem Fall bei der unteren Schnittlinie des vertikalen Querschnitts der Kolonne 1 horizontal ausgerichtet und nicht wie bei dem in Figur 2 gezeigten Ausführungsbeispiel geneigt. Der Stutzen 11 ist zylinderförmig. Eine Polymerisatbildung wird jedoch auch bei diesem Ausführungsbeispiel durch die Sprüheinrichtung 20 verhindert.

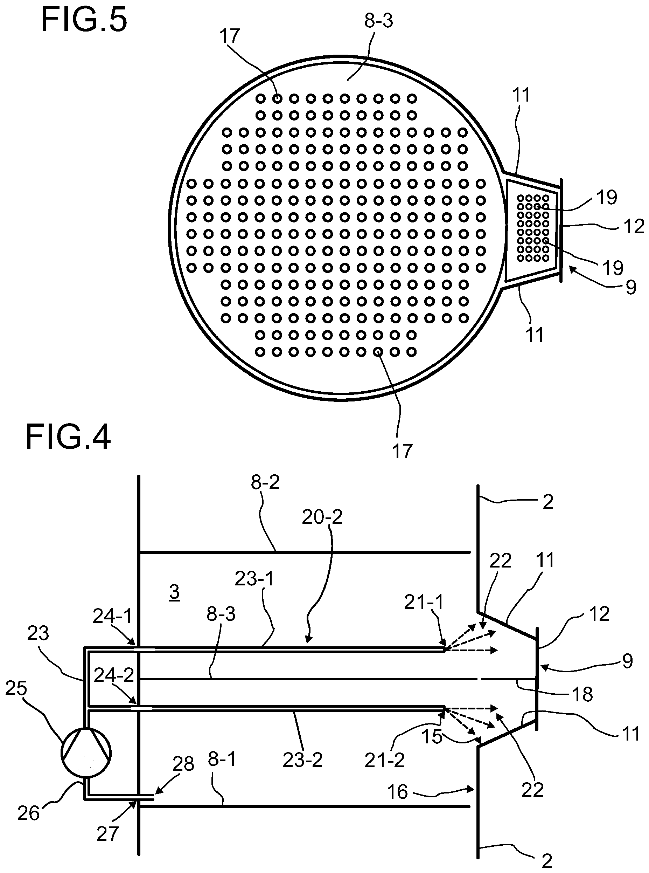

Mit Bezug zu den Figuren 4 und 5 wird ein weiteres Ausführungsbeispiel der erfindungsgemäßen Kolonne 1 beschrieben:
Wie bei dem in den Figuren 1 bis 3 gezeigten Ausführungsbeispiel handelt es sich bei der Revisionsöffnung 9 um eine Mannlochöffnung. Der Abstand zwischen den Stoffaustauschböden 8-1 und 8-2 ist in diesem Fall relativ groß, beispielsweise 1000 mm.

Dieser relativ große Abstand zwischen den beiden Stoffaustauschböden 8-1 und 8-2 kann zu einer unerwünschten Polymerisatbildung führen. Aus diesem Grund ist bei dem Ausführungsbeispiel der Figuren 4 und 5 auch im Bereich der Revisionsöffnung 9 ein Stoffaustauschboden 8-3 angeordnet. Der Abstand zwischen den beiden Stoffaustauschböden 8-1 und 8-3 bzw. zwischen den beiden Stoffaustauschböden 8-3 und 8-2 ist dann 500 mm. Bei dem Stoffaustauschboden 8-3 handelt es sich im beschriebenen Ausführungsbeispiel um einen Dual-Flow-Boden, welcher Öffnungen 17 aufweist, wie es in Figur 5 gezeigt ist.

Des Weiteren ist im Bereich der Revisionsöffnung 9 eine Platte 18 angeordnet, die verhindert, dass insbesondere aufsteigendes Gas, jedoch auch absteigende Flüssigkeit an dem Stoffaustauschboden 8-3 vorbei durch die von dem Stutzen 11 gebildete horizontale Öffnung nach oben strömt bzw. nach unten fließt. Die Platte 18 weist Öffnungen 19 auf, so dass sie als Stoffaustauschplatte wirkt. Die Platte 18 ist horizontal ausgerichtet, wobei sie mit dem Stoffaustauschboden 8-3 fluchtet. Die Platte 18 ist somit horizontal auf derselben Höhe wie der Stoffaustauschboden 8-3 angeordnet. Die Form der Platte 18 ist wie in Figur 3 gezeigt an die horizontale Querschnittsform des Stutzens angepasst. Da der Stutzen im vorliegenden Ausführungsbeispiel kegelstumpfförmig ist, ist die Platte 18 trapezförmig. Um die Spalte bzw. Fuge zwischen der Platte 18 und dem Stoffaustauschboden 8-3 so eng wie möglich zu halten, könnte die lange Seite des Trapezes der Platte 18 auch an die Rundung des Stoffaustauschbodens 8-3 in diesem Bereich angepasst werden oder umgekehrt die Rundung des Stoffaustauschbodens 8-3 in diesem Bereich an die lange Seite der trapezförmigen Platte 18 angepasst abgeflacht sein.

Je nach Größe der Revisionsöffnung 9 und gewünschten Abstand zwischen den Stoffaustauschböden 8 können sich auch mehrere Platten 8 im Bereich der Revisionsöffnung 9 befinden. Diesen Stoffaustauschböden 8 ist dann jeweils eine Platte 18 zugeordnet.

Die Platte 18 ist an dem schwenkbaren Deckel 12 befestigt, so dass sie mit dem Deckel 12 herausgeschwenkt wird, wenn die Revisionsöffnung 9 geöffnet wird. Dies hat den Vorteil, dass die Platte 18 nicht demontiert werden muss, wenn Revisions- oder Reinigungsarbeiten in der Kolonne 1 durchgeführt werden müssen. Gleichermaßen ist auch der Stoffaustauschboden 8-3 demontierbar, so dass eine Person durch die als Mannloch ausgebildete Revisionsöffnung 9 in den Kolonnenhohlraum 3 gelangen kann.

Wie in den Figuren 4 und 5 gezeigt, ist der Stutzen 11 wie bei den vorhergehenden Ausführungsbeispielen kegelstumpfförmig. Alternativ könnte es jedoch auch wie in Figur 3 dargestellt zylindrisch sein.

Die Sprüheinrichtung 20-2 des Ausführungsbeispielen der Figuren 4 und 5 unterscheidet sich von der Sprüheinrichtung 20 der vorstehend beschriebenen Ausführungsbeispiele dadurch, dass sich die Zuleitung 23 in eine obere Zuleitung 23-1 und eine untere Zuleitung 23-2 verzweigt, welche dann durch die Durchführungen 24-1 und 24-2 in den Kolonnenhohlraum 3 eintreten. Die obere Zuleitung 23-1 ist dabei oberhalb des Bodens 8-3 angeordnet, die untere Zuleitung 23-2 hingegen unterhalb des Bodens 8-3. Die obere Zuleitung 23-1 mündet in eine Sprühdüse 21-1, mittels welcher Flüssigkeit 22 gegen die obere Oberfläche 15 des Stutzens 11 sowie die Innenfläche des Deckels 12 gesprüht wird. Die untere Zuleitung 23-2 mündet in eine Sprühdüse 21-2, mittels welcher Flüssigkeit 22 gegen die untere Oberfläche 15 des Stutzens 11 sowie die Innenfläche des Deckels 12 gesprüht wird.

Auch in diesem Ausführungsbeispiel ist außerhalb des Kolonnenkörpers 2 eine Pumpe 25 angeordnet, welche mit der Zuleitung 23 verbunden ist. Auf der anderen Seite ist die Pumpe 25 mit der Leitung 26 verbunden, welche über eine weitere gas- und flüssigkeitsdichte Durchführung 27 wieder in den Kolonnenhohlraum eintritt. Die Einlauföffnung 28 der Leitung 26 ist in diesem Fall unmittelbar oberhalb des Stoffaustauschbodens 8-1 angeordnet ist. Der Stoffaustauschboden 8-1 ist dabei der nächste Stoffaustauschboden unterhalb des Stutzens 11.

Bei weiteren Ausführungsbeispielen kann die Sprüheinrichtung 20 ausgehend von der Zuleitung 23 auch ein Leitungssystem umfassen, welches die Innenoberflächen weiterer Revisionsöffnungen mit Flüssigkeit besprüht. In diesem Fall ist die Zusammensetzung der über die Einlauföffnung 28 entnommenen Flüssigkeit jedoch nicht immer im Wesentlichen dieselbe, wie die Zusammensetzung der Flüssigkeit im Bereich der jeweiligen Revisionsöffnung 9 beim Betrieb eines Trennverfahrens, bei dem insbesondere Gas aufsteigt und eine Flüssigkeit absteigt.

Im Folgenden wird ein Ausführungsbeispiel des erfindungsgemäßen Verfahrens beschrieben, welches mit der vorstehend beschriebenen Trennkolonne 1 ausgeführt wird.

Bei dem Verfahren handelt es sich um ein thermisches Trennverfahren zwischen wenigstens einem in der Trennkolonne 1 aufsteigenden Gas und wenigstens einer in der Trennkolonne 1 absteigenden Flüssigkeit. Dabei enthält das aufsteigende Gas und/oder die absteigende Flüssigkeit insbesondere (Meth)acrylmonomere.

Bei dem Trennverfahren wird eine fraktionierende Kondensation zur Abtrennung von Acrylsäure aus einem Acrylsäure enthaltenden Produktgasgemisch einer heterogen katalysierten Gasphasenpartialoxidation einer C3-Vorläuferverbindung (insbesondere Propen und/oder Propan) der Acrylsäure mit molekularem Sauerstoff zu Acrylsäure in einer trennwirksame Einbauten enthaltenden Trennkolonne 1 durchgeführt. Die Trennkolonne enthält von unten nach oben zunächst Dual-Flow-Böden und im Anschluss daran Querstrom-Haubenböden, die wie vorstehend beschrieben von unten gestützt werden. Ansonsten wird das Verfahren durchgeführt, wie es in den Schriften DE 19924532 A1, DE 10243625 A1 und WO 2008/090190 A1 beschrieben ist.

Unter dem Begriff "C3-Vorläufer" von Acrylsäure werden dabei solche chemischen Verbindungen zusammengefasst, die formal durch Reduktion von Acrylsäure erhältlich sind. Bekannte C3-Vorläufer von Acrylsäure sind z.B. Propan, Propen und Acrolein. Aber auch Verbindungen wie Glyzerin, Propionaldehyd, Propionsäure oder 3-Hydroxypropionsäure sind zu diesen C3-Vorläufern zu zählen. Von ihnen ausgehend handelt es sich bei der heterogen katalysierten Gasphasen-Partialoxidation mit molekularem Sauerstoff wenigstens teilweise um eine oxidative Dehydrierung. Bei den relevanten heterogen katalysierten Gasphasen-Partialoxidationen werden die genannten C3-Vorläufer der Acrylsäure, in der Regel mit inerten Gasen wie z.B. molekularer Stickstoff, CO, CO2, inerte Kohlenwasserstoffe und/oder Wasserdampf verdünnt, im Gemisch mit molekularem Sauerstoff bei erhöhten Temperaturen sowie gegebenenfalls erhöhtem Druck über übergangsmetallische Mischoxidkatalysatoren geleitet und oxidativ in ein Acrylsäure enthaltendes Produktgasgemisch umgewandelt.

In typischer Weise weist das Acrylsäure enthaltende Produktgasgemisch einer heterogen katalysierten Gasphasen-Partialoxidation von C3-Vorläufern (z.B. Propen) der Acrylsäure mit molekularem Sauerstoff an im festen Aggregatzustand befindlichen Katalysatoren, bezogen auf die Gesamtmenge der (in ihm) enthaltenen angegebenen Bestandteile, nachfolgende Gehalte auf:

| |
|---|
| 1 bis 30 Gew.-% Acrylsäure, |
| 0,05 bis 10 Gew.-% molekularer Sauerstoff, |
| 1 bis 30 Gew.-% Wasser, |
| 0 bis 5 Gew.-% Essigsäure, |
| 0 bis 3 Gew.-% Propionsäure, |
| 0 bis 1 Gew.-% Maleinsäure und/oder Maleinsäure-Anhydrid, |
| 0 bis 2 Gew.-% Acrolein, |
| 0 bis 1 Gew.-% Formaldehyd, |
| 0 bis 1 Gew.-% Furfural, |
| 0 bis 0,5 Gew.-% Benzaldehyd, |
| 0 bis 1 Gew.-% Propen, |

und
als Restmenge inerte Gase wie z.B. Stickstoff, Kohlenmonoxid, Kohlendioxid, Methan und/oder Propan.

Die partielle Gasphasenoxidation selbst kann wie im Stand der Technik beschrieben durchgeführt werden. Ausgehend von Propen kann die partielle Gasphasenoxidation z.B. in zwei aufeinanderfolgenden Oxidationsstufen durchgeführt werden, wie sie z.B. in der EP 700 714 A1 und in der EP 700 893 A1 beschrieben sind. Selbstverständlich können aber auch die in der DE 19740253 A1 sowie in der DE 19740252 A1 zitierten Gasphasen-Partialoxidationen zur Anwendung kommen.

In der Regel beträgt die Temperatur des die partielle Gasphasenoxidation verlassenden Produktgasgemischs 150 bis 350°C, häufig 200 bis 300°C.

Durch direkte und/oder indirekte Kühlung wird das heiße Produktgasgemisch zweckmäßigerweise zunächst auf eine Temperatur von 100 bis 180°C abgekühlt, bevor es zum Zweck der fraktionierenden Kondensation in den Bereich C (den Sumpf) der Trennkolonne 1 geführt wird. Der in der Trennkolonne 1 herrschende Betriebsdruck beträgt in der Regel 0,5 bis 5 bar, häufig 0,5 bis 3 bar und vielfach 1 bis 2 bar.

### Bezugszeichenliste

- 1: Kolonne, Trennkolonne
- 2: Kolonnenkörper
- 3: Kolonnenhohlraum
- 4: Zulauf
- 5: Zuleitung
- 6: Ablauf
- 7: Mantel
- 8: Böden
- 8-1, 8-2, 8-3: Böden
- 9: Revisionsöffnung
- 11: Stutzen
- 12: Deckel
- 13: Abzug
- 14: Entnahmeleitung
- 15: Oberfläche
- 16: Innenoberfläche
- 17: Öffnung
- 18: Platte
- 19: Öffnung
- 20: Sprüheinrichtung
- 21: Sprühdüse
- 22: Flüssigkeit
- 23: Zuleitung
- 24: Durchführung
- 25: Pumpe
- 26: Leitung
- 27: Durchführung
- 28: Entnahmeöffnung

## Patentansprüche

1. Kolonne (1) zur thermischen Behandlung von fluiden Gemischen mit
einem zylindrischen, vertikal ausgerichteten Kolonnenkörper (2), der einen Kolonnenhohlraum (3) bildet,
mehreren im Kolonnenhohlraum (3) montierten Böden (8), die vertikal beabstandet voneinander angeordnet sind,
zumindest einem Stutzen (11), der in dem Kolonnenkörper (2) angeordnet ist und der sich von dem Kolonnenkörper (2) weg erstreckt, und
einer verschließbaren Revisionsöffnung (9), die bei dem Stutzen (11) gebildet ist,
wobei in dem Kolonnenkörper (2) eine Sprüheinrichtung (20) angeordnet ist, mit der Flüssigkeit (22) zumindest gegen die in den Kolonnenhohlraum (3) gerichtete Oberfläche (15) des Stutzens (11) sprühbar ist, und
wobei die Sprüheinrichtung (20) eine Sprühdüse (21), eine Zuleitung (23) und eine Sprühflüssigkeits-Zuführeinrichtung aufweist, wobei die Sprühflüssigkeits-Zuführeinrichtung ausgebildet ist, Sprühflüssigkeit dem Kolonnenhohlraum (3) zu entnehmen, die entnommene Sprühflüssigkeit über die Zuleitung (23) der Sprühdüse (21) zuzuführen und mittels der Sprühdüse (21) zumindest gegen die in den Kolonnenhohlraum (3) gerichtete Oberfläche (15) des Stutzens (11) zu sprühen, **dadurch gekennzeichnet, dass** die Sprühflüssigkeits-Zuführeinrichtung eine Einlauföffnung (28) aufweist, die unmittelbar oberhalb eines Bodens (8-1) angeordnet ist, der benachbart zu dem Stutzen (11) ist.

2. Kolonne (1) nach einem der vorhergehenden Ansprüche, **dadurch gekennzeichnet, dass**
der Kolonnenkörper (2) eine vertikale Innenoberfläche (16) bildet und
bei einem vertikalen Querschnitt der Kolonne (1) die in den Kolonnenhohlraum (3) gerichtete Linie der unteren Schnittlinie des Stutzens (11) oder eine Tangente an der in den Kolonnenhohlraum (3) gerichteten Linie der unteren Schnittlinie des Stutzens (11) mit der vertikalen Innenlinie (16) des Kolonnenkörpers (2) zumindest abschnittsweise einen Winkel in einem Bereich von 210° bis 267° einschließt.

3. Kolonne (1) nach Anspruch 2, **dadurch gekennzeichnet, dass**
bei einem vertikalen Querschnitt der Kolonne (1) zumindest 50% der in den Kolonnenhohlraum (3) gerichteten Linie der unteren Schnittlinie des Stutzens (11) oder die Tangente an 50% der in den Kolonnenhohlraum (3) gerichteten Linie der unteren Schnittlinie des Stutzens (11) mit der vertikalen Innenlinie (16) des Kolonnenkörpers (2) einen Winkel in einem Bereich von 225° bis 267° einschließt.

4. Kolonne (1) nach Anspruch 2 oder 3, **dadurch gekennzeichnet, dass**
der Stutzen (11) eine obere Hälfte und eine untere Hälfte aufweist und dass bei der unteren Hälfte die in den Kolonnenhohlraum (3) gerichtete Oberfläche (15) des Stutzens (11) oder die Tangente an der Oberfläche (15) der unteren Hälfte des Stutzens (11) mit der vertikalen Innenoberfläche (16) des Kolonnenkörpers (2) einen Winkel in einem Bereich von 210° und 267° einschließt.

5. Kolonne (1) nach einem der Ansprüche 2 bis 4, **dadurch gekennzeichnet, dass**
der Stutzen (11) um eine horizontale Achse rotationssymmetrisch ausgebildet ist und die in den Kolonnenhohlraum (3) gerichtete Oberfläche (15) des Stutzens (11) oder die Tangente auf der Oberfläche (15) des Stutzens (11) mit der vertikalen Innenoberfläche (16) des Kolonnenkörpers (2) einen Winkel in einem Bereich von 210° bis 267° einschließt.

6. Kolonne (1) nach einem der Ansprüche 2 bis 5, **dadurch gekennzeichnet, dass**
der Stutzen (11) kegelstumpfförmig ausgebildet ist und die in den Kolonnenhohlraum (3) gerichtete Oberfläche (15) des Stutzens (11) mit der vertikalen Innenoberfläche (16) des Kolonnenkörpers (2) einen Winkel in einem Bereich von 210° bis 267° einschließt.

7. Kolonne (1) nach einem der vorhergehenden Ansprüche, **dadurch gekennzeichnet, dass**
die Revisionsöffnung (9) eine Mannlochöffnung ist, die bei dem Stutzen (11) gebildet ist und die mit einem Deckel (12) verschließbar ist,
zumindest einer der Böden (8-3) im Bereich der Mannlochöffnung montiert ist und
im Bereich des Stutzens (11) zwischen dem einen Boden (8-3) und dem geschlossenen Deckel (12) eine Platte (18) angeordnet ist.

8. Kolonne (1) nach Anspruch 7, **dadurch gekennzeichnet, dass**
der gesamte horizontale Querschnitt der Kolonne (1) auf der Höhe des einen Bodens (8-3) im Bereich der Mannlochöffnung von dem einen Boden (8-3) und der Platte (18) im Wesentlichen ausgefüllt ist.

9. Kolonne (1) nach einem der Ansprüche 7 oder 8, **dadurch gekennzeichnet, dass**
der eine Boden (8-3) im Bereich der Mannlochöffnung ein Stoffaustauschboden mit Öffnungen (17) ist und die Platte (18) eine Stoffaustauschplatte mit Öffnungen (19) ist.

10. Kolonne (1) nach einem der Ansprüche 7 bis 9, **dadurch gekennzeichnet, dass**
der eine Boden (8-3) im Bereich der Mannlochöffnung und die Platte (18) im Wesentlichen horizontal ausgerichtet sind.

11. Thermisches Trennverfahren zwischen wenigstens einem in der Kolonne (1) nach einem der Ansprüche 1 bis 10 aufsteigenden Gas und wenigstens einer in der Kolonne (1) absteigenden Flüssigkeit.

12. Verfahren nach Anspruch 11, **dadurch gekennzeichnet, dass**
das aufsteigende Gas und/oder die absteigende Flüssigkeit (Meth)acrylmonomere enthält.

## Claims

1. A column (1) for thermal treatment of fluid mixtures, having
a cylindrical, vertical column body (2) which forms a column cavity (3),
a plurality of trays (8) mounted in the column cavity (3) and spaced apart vertically from one another,
at least one stub (11) disposed within the column body (2) and extending away from the column body (2), and
a closable inspection orifice (9) formed in the stub (11),
where a spray device (20) disposed in the column body (2) can spray liquid (22) at least against the surface (15) of the stub (11) directed into the column cavity (3) and where the spray device (20) has a spray nozzle (21), an inlet (23) and a spray liquid feed device, the spray liquid feed device being designed to draw spray liquid from the column cavity (3), to feed the spray liquid withdrawn through the inlet (23) to the spray nozzle (21) and to spray it by means of the spray nozzle (21) at least against the surface (15) of the stub (11) directed into the column cavity (3), wherein the spray liquid feed device has an intake orifice (28) disposed immediately above a tray (8-1) adjacent to the stub (11).

2. The column (1) according to any of the preceding claims, wherein
the column body (2) forms a vertical inner surface (16) and
in the case of a vertical cross section of the column (1) the line of the lower line of intersection of the stub (11) directed into the column cavity (3) or a tangent to the line of the lower line of intersection of the stub (11) directed into the column cavity (3) at least in sections forms an angle within a range from 210° to 267° with the vertical inner line (16) of the column body (2).

3. The column (1) according to claim 2, wherein
in the case of a vertical cross section of the column (1) at least 50% of the line of the lower line of intersection of the stub (11) directed into the column cavity (3) or the tangent to 50% of the line of the lower line of intersection of the stub (11) directed into the column cavity (3) forms an angle within a range from 225° to 267° with the vertical inner line (16) of the column body (2).

4. The column (1) according to claim 2 or 3, wherein
the stub (11) has an upper half and a lower half and in the lower half the surface (15) of the stub (11) directed into the column cavity (3) or the tangent to the surface (15) of the lower half of the stub (11) forms an angle within a range from 210° to 267° with the vertical inner surface (16) of the column body (2).

5. The column (1) according to any of claims 2 to 4, wherein
the stub (11) is rotationally symmetric about a horizontal axis and the surface (15) of the stub (11) directed into the column cavity (3) or the tangent to the surface (15) of the stub (11) forms an angle within a range from 210° to 267° with the vertical inner surface (16) of the column body (2).

6. The column (1) according to any of claims 2 to 5, wherein
the stub (11) is frustoconical and the surface (15) of the stub (11) directed into the column cavity (3) forms an angle within a range from 210° to 267° with the vertical inner surface (16) of the column body (2).

7. The column (1) according to any of the preceding claims, wherein
the inspection orifice (9) is a manhole orifice which is formed in the stub (11) and can be closed with a cover (12),
at least one of the trays (8-3) is mounted in the region of the manhole orifice and
a plate (18) is disposed in the region of the stub (11) between the one tray (8-3) and the closed cover (12).

8. The column (1) according to claim 7, wherein
the entire horizontal cross section of the column (1) at the height of the one tray (8-3) in the region of the manhole orifice is essentially filled by the one tray (8-3) and the plate (18).

9. The column (1) according to either of claims 7 and 8, wherein
the one tray (8-3) in the region of the manhole orifice is a mass transfer tray having orifices (17) and the plate (18) is a mass transfer plate having orifices (19).

10. The column (1) according to any of claims 7 to 9,
wherein
the one tray (8-3) in the region of the manhole orifice and the plate (18) are aligned essentially horizontally.

11. A thermal separation process between at least one gas ascending within the column (1) according to any of claims 1 to 10 and at least one liquid descending within the column (1).

12. The process according to claim 11, wherein
the ascending gas and/or the descending liquid comprises (meth)acrylic monomers.

## Revendications

1. Colonne (1) destinée au traitement thermique de mélanges fluides, ladite colonne comprenant
un corps de colonne cylindrique (2) orienté verticalement et formant une cavité de colonne (3),
une pluralité de plateaux (8) montés dans la cavité de colonne (3) et espacés verticalement les uns des autres,
au moins une tubulure (11) qui est disposée dans le corps de colonne (2) et qui s'étend depuis le corps de colonne (2), et
une ouverture d'inspection fermable (9) qui est ménagée dans la tubulure (11),
un dispositif de pulvérisation (20) étant disposé dans le corps de colonne (2) et permettant de pulvériser du liquide (22) au moins contre la surface (15), dirigée dans la cavité de colonne (3), de la tubulure (11), et
le dispositif de pulvérisation (20) comportant une buse de pulvérisation (21), une conduite d'alimentation (23) et un dispositif d'alimentation en liquide de pulvérisation, le dispositif d'alimentation en liquide de pulvérisation étant conçu pour retirer le liquide de pulvérisation de la cavité de colonne (3), pour amener le liquide de pulvérisation retiré à la buse de pulvérisation (21) par le biais de la conduite d'alimentation (23) et pour la pulvériser au moyen de la buse de pulvérisation (21) au moins contre la surface (15), dirigée dans la cavité de colonne (3), de la tubulure (11), **caractérisée en ce que** le dispositif d'alimentation en liquide de pulvérisation comporte une ouverture d'entrée (28) qui est disposé immédiatement au-dessus d'un plateau (8-1) qui est adjacent à la tubulure (11).

2. Colonne (1) selon l'une des revendications précédentes, **caractérisée en ce que**
le corps de colonne (2) forme une surface intérieure verticale (16) et
dans le cas d'une section verticale de la colonne (1) la ligne, dirigée dans la cavité de la colonne (3), de la ligne de coupe inférieure de la tubulure (11) ou une tangente à la ligne, dirigée dans la cavité de colonne (3), de la ligne de coupe inférieure de la tubulure (11) forme avec la ligne intérieure verticale (16) du corps de colonne (2) au moins en par endroits un angle dans une plage de 210° à 267°.

3. Colonne (1) selon la revendication 2, **caractérisée en ce que**
dans le cas d'une section verticale de la colonne (1) au moins 50 % de la ligne, dirigée dans la cavité de colonne (3), de la ligne de coupe inférieure de la tubulure (11) ou la tangente à 50 % de la ligne, dirigée dans la cavité de colonne (3), de la ligne de coupe inférieure de la tubulure (11) forme avec la ligne intérieure verticale (16) du corps de colonne (2) un angle dans une plage de 225° à 267°.

4. Colonne (1) selon la revendication 2 ou 3, **caractérisée en ce que**
la tubulure (11) comporte une moitié supérieure et une moitié inférieure et **en ce que** dans la moitié inférieure la surface (15), dirigée dans la cavité de colonne (3), de la tubulure (11) ou la tangente à la surface (15) de la moitié inférieure de la tubulure (11) forme avec la surface intérieure verticale (16) du corps de colonne (2) un angle dans une plage de 210° et 267°.

5. Colonne (1) selon l'une des revendications 2 à 4, **caractérisée en ce que**
la tubulure (11) est conçue à symétrie de révolution sur un axe horizontal et la surface (15), dirigée dans la cavité de colonne (3), de la tubulure (11) ou la tangente à la surface (15) de la tubulure (11) forme avec la surface intérieure verticale (16) du corps de colonne (2) un angle dans une plage de 210° à 267°.

6. Colonne (1) selon l'une des revendications 2 à 5, **caractérisée en ce que**
la tubulure (11) est tronconique et la surface (15), dirigée dans la cavité de colonne (3), de la tubulure (11) forme avec la surface intérieure verticale (16) du corps de colonne (2) un angle dans une plage de 210° à 267°.

7. Colonne (1) selon l'une des revendications précédentes, **caractérisée en ce que**
l'ouverture de contrôle (9) est une ouverture en trou d'homme qui est formée dans la tubulure (11) et qui peut être fermée avec un couvercle (12),
au moins l'un des plateaux (8-3) est monté dans la région de l'ouverture en tour d'homme et
une plaque (18) est disposée dans la région de la tubulure (11) entre l'un des plateaux (8-3) et le couvercle fermé (12).

8. Colonne (1) selon la revendication 7, **caractérisée en ce que**
toute la section horizontale de la colonne (1) au niveau de l'un des plateaux (8-3) dans la région de l'ouverture en trou d'homme est sensiblement remplie par l'un des plateaux (8-3) et la plaque (18).

9. Colonne (1) selon l'une des revendications 7 ou 8, **caractérisée en ce que**
l'un des plateaux (8-3) dans la région de l'ouverture en trou d'homme est un plateau d'échange de matière pourvu d'ouvertures (17) et la plaque (18) est une plaque d'échange de matière pourvue d'ouvertures (19) .

10. Colonne (1) selon l'une des revendications 7 à 9, **caractérisée en ce que**
l'un des plateaux (8-3) dans la région de l'ouverture en trou d'homme et la plaque (18) sont orientés sensiblement horizontalement.

11. Procédé de séparation thermique entre au moins un gaz montant dans la colonne (1) selon l'une des revendications 1 à 10 et au moins un liquide descendant dans la colonne (1).

12. Procédé selon la revendication 11, **caractérisé en ce que**
le gaz montant et/ou le liquide descendant contiennent des monomères (méth)acryliques.
